# EUROPEAN PATENT SPECIFICATION

(11) **EP 4 252 626 B1**
(45) Date of publication and mention of the grant of the patent: **11.06.2025**
(21) Application number: 23164941.9
(22) Date of filing: 29.03.2023
(51) Int. Cl.: A61B 3/103

(54) **OPHTHALMIC APPARATUS**
OPHTHALMISCHE VORRICHTUNG
APPAREIL OPHTALMIQUE

(30) Priority: 31.03.2022 JP 2022059854; 31.03.2022 JP 2022059855
(43) Date of publication of application: 04.10.2023
(73) Proprietor: NIDEK CO., LTD., Gamagori, Aichi (JP)
(72) Inventor: TAKII, Michihiro, Gamagori, Aichi (JP)
(74) Representative: Hoefer & Partner Patentanwälte mbB

(56) References cited:
- US-A1- 2014 111 769
- US-A1- 2021 330 185

## Description

### TECHNICAL FIELD

The present disclosure relates to an ophthalmic apparatus that objectively measures optical characteristics of a subject eye.

### BACKGROUND

An ophthalmic apparatus is known that objectively measures optical characteristics of a subject eye by projecting a measurement light flux toward the fundus of the subject eye and detecting a reflection light flux reflected by the fundus (for example, refer to JP2006-187483A). For example, in a wavefront aberration measurement apparatus disclosed in JP2010-099355A, a Hartmann plate is used to divide a reflection light flux into a plurality of light fluxes, and the wavefront aberration is measured based on the state of separation of images thereof (for example, refer to JP2010-099355A).

The first issue will be explained. In recent years, it is required to improve accuracy in measuring optical characteristics of an intraocular lens implanted eye. However, the wavefront aberration measurement apparatus of JP2010-099355Amay not be able to obtain measurement results with high accuracy because it is difficult to separate images due to the difference in the characteristics of the intraocular lens. In addition, the wavefront aberration measurement apparatus of JP2010-099355Adoes not get the characteristics of the intraocular lens inserted into the subject eye, and it is considered that the apparatus configuration needs to be complicated in order to determine the characteristics.

The second issue will be described. For example, when a monofocal intraocular lens, a multifocal intraocular lens, an expanded depth of field (EDoF) intraocular lens, or the like is inserted into the subject eye, due to the difference in characteristics of each intraocular lens, it may be difficult to appropriately measure the optical characteristics. Furthermore, since the characteristics of the intraocular lens inserted into the subject eye are unknown, it may be difficult to determine whether the measurement result is appropriate.
US 2014 / 111 769 A1 discloses a compensation optical apparatus with the goal of obtaining an image of an object without reduction in image quality irrespective of aberration compensation, including: a division unit for dividing a return beam from a measured object; an aberration measurement unit for measuring an aberration caused by the measured object, with a divided beam from the division unit; an aberration compensation unit for performing aberration compensation based on the aberration measured by the aberration measurement unit; a projection unit for projecting a beam obtained by the aberration compensation in the aberration compensation unit to the measured object; an acquirement unit for acquiring a value exhibiting a state of the measured object based on the return beam from the measured object, which is obtained by the beam projected from the projection unit; and a control unit for retreating the division unit from an optical path based on the value acquired by the acquirement unit.

### SUMMARY

A typical object of the present disclosure is to provide an ophthalmic apparatus capable of measuring optical characteristics of an intraocular lens implanted eye with a simple configuration. Another typical object of the present disclosure is to provide an ophthalmic apparatus capable of satisfactorily measuring the optical characteristics of the intraocular lens implanted eye.
(1) An ophthalmic apparatus that objectively measures optical characteristics of a subject eye, having:
   an eye refractive power measuring optical system including a first light projecting optical system that projects a first measurement light flux toward a fundus of the subject eye, and a first light receiving optical system in which a first detector detects a first reflection light flux, reflected by the fundus, of the first measurement light flux as a first index pattern image, and for acquiring an eye refractive power of the subject eye; and
   a contrast measuring optical system including a second light projecting optical system that projects a patterned second measurement light flux toward the fundus of the subject eye, and a second light receiving optical system in which a second detector detects a second index pattern image formed by a second reflection light flux, reflected by the fundus, of the second measurement light flux, and for acquiring contrast information related to a contrast of the subject eye,
   in which the first detector and the second detector are different detectors.
(2) The ophthalmic apparatus according to the above-described (1),
   in which in the first light receiving optical system, the first detector detects the first reflection light flux, which passes through a pupil divided region formed by dividing a pupil region of the subject eye, as the first index pattern image, and
   in the second light receiving optical system, the second detector detects the second index pattern image formed by the second reflection light flux which passes through the pupil region of the subject eye.
(3) The ophthalmic apparatus according to the above-described (1) or (2),
   in which the first light receiving optical system has a pattern optical member configured to condense the first reflection light flux from the fundus as a pattern light flux on
   the first detector, and the pattern optical member causes the first detector to detect the first index pattern image.
(4) The ophthalmic apparatus of the above-described (3),
   in which the pattern optical member is a ring optical member configured to condense the first reflection light flux from the fundus as a ring light flux on the first detector, and the ring optical member causes the first detector to detect a ring image as the first index pattern image.
(5) The ophthalmic apparatus according to the above-described (3) or (4), further having:
   a first optical path branching member configured to branch an optical path of the first light projecting optical system and an optical path of the first light receiving optical system; and
   a second optical path branching member configured to branch an optical path of the first light receiving optical system and an optical path of the second light receiving optical system,
   in which the second optical path branching member is arranged between the first optical path branching member and the pattern optical member to dispose the first detector and the second detector in different optical paths.
(6) The ophthalmic apparatus according to any one of the above-described (1) to (5),
   in which the first measurement light flux is an infrared light flux and the second measurement light flux is a visible light flux.
(7) The ophthalmic apparatus according to any one of the above-described (1) to (6), further having:
   a first acquisition means configured to project the first measurement light flux toward the fundus of the subject eye, and cause the first detector to detect the first reflection light flux, reflected by the fundus, of the first measurement light flux, to acquire optical characteristics of the subject eye;
   a second acquisition means configured to project the second measurement light flux toward the fundus of the subject eye, and cause the second detector to detect the second reflection light flux, reflected by the fundus, of the second measurement light flux, to acquire a plurality of contrast information with at least different diopter information in the subject eye; and
   an output means configured to output change information of the contrast information with respect to the diopter information.
(8) The ophthalmic apparatus according to the above-described (7),
   in which the change information includes first data indicating a peak value of the contrast information with respect to the diopter information.
(9) The ophthalmic apparatus according to the above-described (8),
   in which the change information includes second data indicating a peripheral value for the first data.
(10) The ophthalmic apparatus according to any one of the above-described (7) to (9),
   in which the output means is configured to output, as the change information, a graph showing a distribution of the contrast information with respect to the diopter information.
(11) The ophthalmic apparatus according to the above-described (10),
   in which the output means is configured to output, as the change information, the graph continuously showing a change in the contrast information accompanied by a change in the diopter information.
(12) The ophthalmic apparatus according to any one of the above-described (7) to (10),
   in which the second acquisition means is configured to acquire the contrast information with different diopter information of the subject eye, based on the optical characteristics of the subject eye acquired by the first acquisition means.
(13) The ophthalmic apparatus according to any one of the above-described (7) to (11),
   in which the second acquisition means is configured to project the patterned light flux as the second measurement light flux, cause the second detector to detect the second index pattern image changing periodically and formed by the second reflection light flux, and acquire the contrast information based on luminance information in the second index pattern image.
(14) The ophthalmic apparatus according to any one of the above-described (7) to (13), further having:
   a correction means configured to correct the optical characteristics acquired by the first acquisition means, based on the contrast information acquired by the second acquisition means.
(15) An ophthalmic apparatus that objectively measures optical characteristics of a subject eye, having:
   a first acquisition means configured to project a first measurement light flux toward a fundus of the subject eye, and cause a first detector to detect a first reflection light flux, reflected by the fundus, of the first measurement light flux, to acquire optical characteristics of the subject eye;
   a second acquisition means configured to project a second measurement light flux toward the fundus of the subject eye, and cause a second detector to detect a second reflection light flux, reflected by the fundus, of the second measurement light flux, to acquire a plurality of contrast information with at least different diopter information in the subject eye; and
   an output means configured to output change information of the contrast information with respect to the diopter information.

### BRIEF DESCRIPTION OF DRAWINGS

FIG. 1 is an external view of an ophthalmic apparatus.
FIG. 2 is a schematic view of an optical system of the ophthalmic apparatus.
FIGs. 3A and 3B are schematic diagrams showing reflection light flux from the fundus of a subject eye and a pupil region.
FIG. 4 is an example of a pattern target plate.
FIG. 5 is a schematic view of a control system of the ophthalmic apparatus.
FIG. 6 is an example of a pattern target image.
FIG. 7 is an example of contrast value change information with respect to diopter information of the subject eye.
FIGs. 8A to 8D are examples of a graph corresponding to the subject eye.
FIG. 9 is an example in which a peak value appears at a distance different from a spherical power of 0D.
FIG. 10 is an example of a graph showing a relationship between a spherical power of the subject eye and a peak value P of the contrast value in each direction.

### DETAILED DESCRIPTION

### <Overview>

An overview of an ophthalmic apparatus according to an embodiment of the present disclosure will be described. The items classified by < > below can be used independently or in association with each other. In the present embodiment, "conjugated" is not necessarily limited to a perfect conjugated relationship, but includes "substantially conjugated". That is, the term "conjugated" in the present embodiment also includes the case where the units are displaced from the perfectly conjugated position within the range allowed in relation to the technical significance of each unit.

The ophthalmic apparatus of the present embodiment is an apparatus capable of objectively acquiring the optical characteristics of a subject eye. For example, the ophthalmic apparatus may have an optical system that is used to measure the optical characteristics of the subject eye. As an example, the ophthalmic apparatus may have an optical system used to measure at least one of aberration amount, eye refractive power (at least one of spherical power, cylindrical power, astigmatism axis angle, and the like), and the like.

For example, the ophthalmic apparatus may have an optical system that is used to acquire contrast information of the subject eye. For example, contrast information may be at least one of a parameter indicating a contrast value, a parameter correlated with the contrast value, a parameter that can be converted into a contrast value, and the like. As an example, contrast information may be a contrast value, a contrast sensitivity, a modulation transfer function (MTF), a point spread function (PSF), or a blurring amount. Contrast information may be a combination of these information. It is needless to say that information different from these may be included.

In addition, contrast information may be information having directionality corresponding to the axis of the subject eye (in other words, the astigmatism axis angle). In this case, one or more contrast information may be acquired according to one or more astigmatism axis angles.

### <Eye refractive power measuring optical system>

The ophthalmic apparatus of the present embodiment may include an eye refractive power measuring optical system (for example, a first measurement optical system 100). The eye refractive power measuring optical system is an optical system for acquiring the eye refractive power of the subject eye. For example, a configuration may be provided for projecting a measurement light flux onto the fundus of the subject eye and acquiring the eye refractive power based on the reflection light flux, which is reflected by the fundus, of the measurement light flux.

More specifically, the eye refractive power measuring optical system may include a first light projecting optical system (for example, a projection optical system 110) that projects a first measurement light flux toward the fundus of the subject eye, and a first light receiving optical system (for example, a light receiving optical system 120) in which a first detector detects a first reflection light flux, reflected by the fundus, of the first measurement light flux. In the first light projecting optical system, the first measurement light flux may be a visible light flux or an infrared light flux. In the first light receiving optical system, the first detector may detect the first reflection light flux as the first index pattern image. In addition, in the first light receiving optical system, the first detector (for example, an imaging element 126) may detect the first reflection light flux, which has passed through a pupil divided region formed by dividing a pupil region of the subject eye, as a first index pattern image.

The first light receiving optical system may have a pattern optical member
configured to condense the first reflection light flux from the fundus as a pattern light flux on the first detector. The pattern optical member causes the first detector to detect the first index pattern image. For example, the pattern optical member may be an optical member capable of converting the first reflection light flux into any pattern light flux. As an example, the pattern optical member may be a screen with multiple holes (so-called Hartmann plate). In this case, a plurality of point images are detected as the first index pattern image. In addition, as an example, the pattern optical member may be a ring optical member. In this case, a ring image is detected as the first index pattern image.

In the present example, the pattern optical member may be a ring optical member for condensing the first reflection light flux from the fundus as a ring light flux on the first detector. The ring optical member may cause the first detector to detect at least one ring image as the first index pattern image. For example, the ring optical member may be a ring lens. As an example, a ring lens having a ring opening in which one or a plurality of rings having different diameters are formed and an annular lens unit corresponding to this ring opening may be used. In addition, instead of the ring lens, a condensing lens and a plurality of conical prisms arranged concentrically may be used.

The ophthalmic apparatus of the present embodiment may include a contrast measuring optical system (for example, a second measurement optical system 300). The contrast measuring optical system is an optical system for acquiring contrast information of a subject eye. For example, a configuration may be provided for projecting a measurement light flux toward the fundus of the subject eye and acquiring contrast information based on a reflection light flux, which is reflected by the fundus, of the measurement light flux.

More specifically, the contrast measuring optical system may include a second light projecting optical system (for example, a light projecting optical system 310) that projects a second measurement light flux toward the fundus of the subject eye, and a second light receiving optical system (for example, a light receiving optical system 320) in which a second detector detects a second reflection light flux, reflected by the fundus, of the second measurement light flux. In the second light projecting optical system, the second measurement light flux may be a visible light flux or an infrared light flux. In addition, the second light projecting optical system may project the patterned second measurement light flux toward the fundus of the subject eye. In the second light receiving optical system, the second detector may detect the second index pattern image formed by the second reflection light flux, reflected by the fundus, of the second measurement light flux. In the second light receiving optical system, the second detector (for example, an imaging element 322) may detect the second index pattern image formed by the second reflection light flux that has passed through the pupil region of the subject eye.

### <First measurement light flux and second measurement light flux>

In the present embodiment, the first measurement light flux of the eye refractive power measuring optical system may be composed of an infrared light flux having a wavelength in the infrared region. For example, the peak wavelength of the first measurement light flux may be between 800 nm and 900 nm. As a result, the examinee is less likely to feel glare when the first measurement light flux is projected, miosis is suppressed, and the eye refractive power can be obtained with high accuracy. However, when the subject eye E is an intraocular lens implanted eye, depending on the type of intraocular lens (for example, monofocal intraocular lens, EDoF type intraocular lens, and multifocal intraocular lens), there is a possibility that the eye refractive power cannot be measured correctly. On the other hand, in the present embodiment, the second measurement light flux of the contrast measuring optical system may be composed of a visible light flux having a wavelength in the visible range. For example, the peak wavelength of the second measurement light flux may be between 400 nm and 800 mm. Accordingly, regardless of whether the subject eye is an intraocular lens implanted eye, contrast information can be acquired satisfactorily. Note that, for example, even when the subject eye is a refractive type intraocular lens implanted eye, contrast information can be acquired because a visible light flux can be appropriately distributed to distant and near areas according to the designed lens characteristics.

In the present embodiment, the first measurement light flux of the eye refractive power measuring optical system may be a spot-shaped light flux. In this case, the spot-shaped light flux in the first light projecting optical system may be detected by the first detector as the first index pattern image through the above-mentioned pattern optical member. Further, in the present embodiment, the first measurement light flux of the eye refractive power measuring optical system may be a patterned light flux. For example, the light flux may be at least one patterned light flux such as point group, linear, radial, or the like. In this case, the patterned light flux in the first light projecting optical system may be detected by the first detector as the first index pattern image without passing through the above-mentioned pattern optical member.

In the present embodiment, the second measurement light flux of the contrast measuring optical system may be a spot-shaped light flux. In this case, the spot-shaped light flux in the second light projecting optical system may be detected by the second detector. Further, in the present embodiment, the second measurement light flux of the contrast measuring optical system may be a patterned light flux. For example, the light flux may be at least one patterned light flux such as point group, linear, radial, or the like. Alternatively, for example, the light flux may be a patterned light flux that changes periodically. As an example, the light flux may be a light flux of which luminance alternately increases and decreases along at least one direction, or a light flux of which luminance changes according to a sine wave function (using a so-called sine wave grating). In these cases, the patterned light flux in the second light projecting optical system may be detected by the second detector as the second index pattern image.

### <Common use of eye refractive power measuring optical system and contrast measuring optical system>

In the present embodiment, the eye refractive power measuring optical system and the contrast measuring optical system may be a common optical path. For example, at least a part of the light projecting optical path of the first measurement light flux in the first light projecting optical system and the light projecting optical path of the second measurement light flux in the second light projecting optical system may be a common optical path. Further, for example, at least a part of the light receiving optical path of the first reflection light flux in the first light receiving optical system and the light receiving optical path of the second reflection light flux in the second light receiving optical system may be a common optical path. It is needless to say that both the common use of the respective light projecting optical paths and the common use of the respective light receiving optical paths may be achieved.

For example, the first light source in the first light projecting optical system and the second light source in the second light projecting optical system may be the same (common) light source. In addition, for example, the first light source in the first light projecting optical system and the second light source in the second light projecting optical system may be different light sources. That is, the light projecting optical path of the first light source in the first light projecting optical system and the light projecting optical path of the second light source in the second light projecting optical system may be coupled in the middle to form a common optical path.

For example, the first detector in the first light receiving optical system and the second detector in the second light receiving optical system may be the same (common) detector. Further, for example, the first detector in the first light receiving optical system and the second detector in the second light receiving optical system may be different detectors. That is, the common optical path in which the light receiving optical path of the first light receiving optical system and the light receiving optical path of the second light receiving optical system are shared may be branched in the middle and guided to either the first detector or the second detector. Accordingly, it is possible to acquire the eye refractive power and contrast information of the subject eye without complicating each optical system.

In the present embodiment, the light projecting optical path of the first light projecting optical system and the light receiving optical path of the first light receiving optical system may be branched by a first optical path branching member. Further, in the present embodiment, the light receiving optical path of the first light receiving optical system and the light receiving optical path of the second light receiving optical system may be branched by a second optical path branching member. For example, the first optical path branching member and the second optical path branching member may be composed of at least one optical member such as a beam splitter, a dichroic mirror, a half mirror, or the like. The second optical path branching member may be arranged between the first optical path branching member and the pattern optical member to dispose the first detector and the second detector in different optical paths.

In addition, when the second reflection light flux of the second light receiving optical system is configured to pass through the pattern optical member as in the first light receiving optical system (that is, when the first detector and the second detector are used together), a second index pattern image formed by the second reflection light flux is divided into a plurality of images by the pattern optical member. As an example, when the pattern optical member is a Hartmann plate, the second index pattern images are separated by the number of holes and detected by the detector as a plurality of images. When the pattern optical member is a ring optical member, a plurality of images are overlapped in a ring shape and detected by the detector. Therefore, it becomes difficult to acquire contrast information. However, by providing the first detector and the second detector respectively as in the present example, the second index pattern image can be detected as one image, and the contrast information can be acquired appropriately.

### <First acquisition means>

The ophthalmic apparatus of the present embodiment may include a first acquisition means (for example, a control unit 80). The first acquisition means projects the first measurement light flux toward the fundus of the subject eye, and causes the first detector to detect the reflection light flux, reflected by the fundus, of the first measurement light flux, to acquire optical characteristics of the subject eye. For example, the eye refractive power of the subject eye may be acquired by analyzing the detection result of the first detector by the eye refractive power measuring optical system. The detection result of the first detector may be signal data or image data.

### <Second acquisition means>

The ophthalmic apparatus of the present embodiment may include a second acquisition means (for example, a control unit 80). The second acquisition means projects the second measurement light flux toward the fundus of the subject eye, and causes the second detector to detect the reflection light flux, reflected by the fundus, of the second measurement light flux, to acquire contrast information of the subject eye. For example, the contrast information of the subject eye may be acquired by analyzing the detection result of the second detector by the contrast measuring optical system. The detection result of the second detector may be signal data or image data.

The second acquisition means may acquire a plurality of contrast information with at least different diopter information in the subject eye. For example, the diopter information may be spherical power information, cylindrical power information, or astigmatism axis angle information. It is needless to say that a combination of these information may also be used.

The second acquisition means may acquire a plurality of contrast information with different diopter information based on the presentation distance (presentation position) of the target presented to the subject eye. In this case, the diopter information may be information obtained by replacing the presentation distance of the target to be presented to the subject eye with diopter information. More specifically, by changing the presentation distance of the target presented to the subject eye, the dioptric power (ocular refraction) of the subject eye may be changed, and the accompanying change in the focal length of the subject eye can be used as the change in diopter information.

The second acquisition means may acquire a plurality of contrast information with different diopter information based on the optical characteristics of the subject eye acquired by the first acquisition means. In this case, the optical characteristics of the subject eye may be used as the diopter information of the subject eye.

Note that the ophthalmic apparatus of the present embodiment may include a diopter information acquisition means (for example, a control unit 80). The diopter information acquisition means acquires diopter information. The second acquisition means may acquire a plurality of contrast information with at least different diopter information in the subject eye based on the diopter information acquired by the diopter information acquisition means.

For example, the diopter information acquisition means may acquire diopter information according to the presentation distance of the target presented to the subject eye. As an example, the diopter information acquisition means may acquire diopter information corresponding to each presentation distance when the target presented to the subject eye is changed between the far distance and the near distance. In this case, the diopter information may be acquired by referring to a correspondence table in which the target presentation distance and the diopter information are associated in advance. Further, in this case, the diopter information may be acquired using an arithmetic expression for replacing the target presentation distance with the diopter information.

For example, the diopter information acquisition means may acquire diopter information of the subject eye. As an example, the diopter information acquisition means may acquire, as the diopter information, each dioptric power when the optical characteristics added to the subject eye are changed. In this case, the diopter information may be acquired using the first acquisition means. That is, the optical characteristics of the subject eye acquired by the first acquisition means may be acquired as the diopter information of the subject eye. Further, in this case, the optical characteristics of the subject eye may be stored in advance in a storage means (for example, a memory 85), and the diopter information of the subject eye may be acquired by calling this. Further, in this case, the diopter information of the subject eye may be acquired by receiving the optical characteristics of the subject eye from another apparatus (another control means).

The second acquisition means may project a patterned light flux as a second measurement light flux, cause the second detector to detect a second index pattern image changes periodically and formed by the second reflection light flux, and acquire contrast information based on luminance information in the second index pattern image. For example, contrast information may be acquired based on luminance information along the direction in which the second index pattern image periodically changes. When the second index pattern image has a plurality of periodically changing directions, contrast information may be acquired based on a plurality of luminance information in each direction. Note that, for example, the luminance information may be information related to luminance. For example, the luminance information may be at least one of information such as the maximum value of luminance, the minimum value of luminance, the ratio of light and shade of luminance, the gradation of luminance, the distribution of luminance, and the like.

### <Correction means>

The ophthalmic apparatus of the present embodiment may include a correction means (for example, a control unit 80). The correction means corrects the optical characteristics acquired by the first acquisition means based on the contrast information acquired by the second acquisition means. For example, the eye refractive power acquired by the first acquisition means may be corrected based on the contrast information acquired by the second acquisition means. For example, by detecting the deviation amount of diopter information between the contrast information acquired by the second acquisition means and the optical characteristic acquired by the first acquisition means, and adding or subtracting the deviation amount to and from the optical characteristics, the optical characteristic may be corrected. As a result, for example, even when the subject eye is an intraocular lens implanted eye and the eye refractive power of the subject eye is not appropriately acquired, the accuracy of the measurement result can be improved by correcting the eye refractive power.

### <Output means>

The ophthalmic apparatus of the present embodiment may include an output means (for example, a control unit 80). The output means outputs change information of the contrast information with respect to the diopter information. For example, the output means may output the change information by at least one of output by displaying the change information on a display means (for example, a display unit 16), output by transmitting the change information to a memory, a server, or the like, output by printing the change information to a printer or the like, output by transmitting these to the external device, and the like. For example, by outputting the change information, it becomes easier to understand whether the subject eye is an intraocular lens implanted eye, and when the subject eye is the intraocular lens implanted eye, it becomes easier to understand what type of characteristics the lens has, and the like. In addition, it becomes easier to determine whether the optical characteristics acquired by the first acquisition means are appropriate.

The change information may include first data indicating a peak value of contrast information with respect to diopter information. For example, the first data may be the peak value of the contrast information, that is, the data corresponding to the apex of the peak. In this case, the data corresponding to the apex may be at least one of the maximum value and a value different from the maximum value.

The change information may include second data indicating the peripheral value for the first data. For example, the second data may include at least one contrast information value at a position where the diopter information is greater than that of the first data, with the diopter information of the first data as a reference. In addition, the second data may include at least one contrast information value at a position where the diopter information is less than that of the first data, with the diopter information of the first data as a reference. In addition, the second data may include a plurality of contrast information values at a position where the diopter information is greater than that of the first data and is less than that of the first data, with the diopter information of the first data as a reference.

The change information may be information capable of representing at least a partial shape of the peak of the contrast information with respect to the diopter information by including both the first data and the second data.

The output means may output the change information such that the first data indicating the peak value of the contrast information with respect to the diopter information and the second data indicating the peripheral value for the first data can be relatively compared. For example, the first data and the second data may be superimposed and output. In addition, for example, the first data and the second data may be output in parallel. Further, for example, the first data and the second data may be output in a switchable manner. It is needless to say that these combinations may allow relative comparison of each data.

The output means may output, as change information, a list in which diopter information and contrast information are associated with each other. For example, a list in which diopter information and a plurality of contrast information including at least the first data are associated with each other may be output. Further, for example, a list in which diopter information and a plurality of contrast information including at least the first data and the second data are associated with each other may be output.

The output means may output the distribution of the contrast information with respect to the diopter information as a graph as the change information. For example, a graph obtained by plotting a plurality of contrast information including at least the first data with respect to diopter information may be output. In addition, for example, a graph obtained by plotting a plurality of contrast information including at least the first data and the second data with respect to diopter information may be output. Accordingly, for example, the contrast information of the subject eye is expressed visually in an easy -to-understand manner. In addition, it is possible to easily understand how the contrast information of the subject eye changes.

The output means may output, as change information, a graph continuously showing a change in contrast information accompanied by a change in diopter information. In other words, a graph that continuously represents a change in contrast information with respect to a change in diopter information may be output. For example, at least the first data may be included in such a continuous change of contrast information. In addition, for example, at least first data and second data may be included. As a result, for example, the positions of the far point and the near point of the subject eye, changes in diopter information before and after the far point and the near point, and the like can be understood.

The output means may output both the optical characteristics of the subject eye acquired by the first acquisition means and the contrast information of the subject eye acquired by the second acquisition means. In this case, the value acquired by the first acquisition means may be output as it is as the optical characteristics of the subject eye. Further, in this case, as the optical characteristics of the subject eye, a value corrected by the correction means may be output. Further, in this case, both the value acquired by the first acquisition means and the value corrected by the correction means may be output as the optical characteristics of the subject eye.

The ophthalmic apparatus of the present embodiment indicates the relationship between diopter information and contrast information for an intraocular lens implanted eye, and accordingly, it is possible to make appropriate prescriptions based on the characteristics of the intraocular lens of the subject eye. For example, when spectacle prescriptions or the like are required for the subject eye E, the calibration amount can be determined in consideration of such information. In addition, for example, when the subject eye needs to newly add an intraocular lens or change the intraocular lens, it is possible to select an appropriate lens.

Note that the ophthalmic apparatus of the present embodiment is not limited to the configuration for acquiring contrast information for an intraocular lens implanted eye. It is needless to say that the configuration may also be employed in which contrast information is similarly acquired for an eye without an intraocular lens. For example, even in an eye without an intraocular lens, there is a possibility that the results of the optical characteristics acquired by the first acquisition means may not be appropriate due to differences in the pupil region of the subject eye (for example, eyes with small pupils). Therefore, an error in optical characteristics may be detected by acquiring contrast information of the subject eye.

### <Example>

An example of the ophthalmic apparatus according to the present embodiment will be described.

### <Overall configuration>

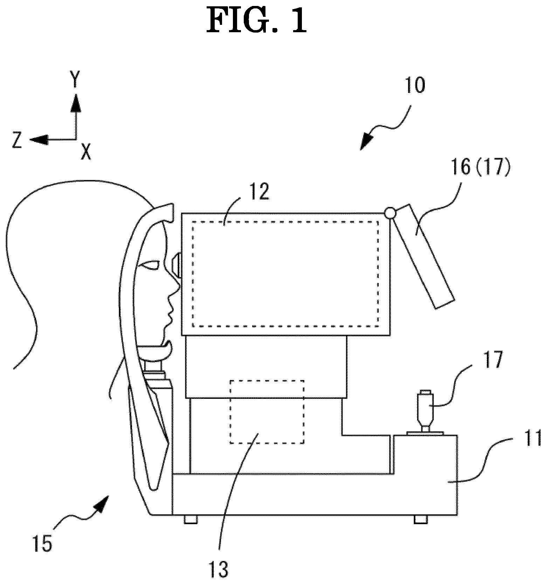

FIG. 1 is an external view of an ophthalmic apparatus 10. The ophthalmic apparatus 10 of the present example is an objective eye refractive power measurement apparatus. Here, an auto refractometer is exemplified. Although the ophthalmic apparatus 10 is a stationary type, the ophthalmic apparatus 10 is not necessarily limited to this, and may be a hand-held type.

The ophthalmic apparatus 10 includes at least a base 11, a measurement unit 12, an alignment driving unit 13, a face support unit 15, a display unit 16, an operation unit 17, a control unit 80, and the like.

The measurement unit 12 accommodates an optical system and the like used for measuring the subject eye. The alignment driving unit 13 can three-dimensionally move the measurement unit 12 with respect to the base 11. The face support unit 15 is fixed to the base 11 and supports the face of the examinee. The display unit 16 functions as a touch panel that also serves as an operation unit. In addition, the display unit 16 displays the eye refractive power, the anterior eye part cross-sectional image, the eye axial length, and the like of the subject eye E on the screen. The display unit 16 displays various types of information (for example, the anterior eye part image of the subject eye and the measurement results of the subject eye). The operation unit 17 performs various settings. Note that the display unit 16 may function as a touch panel, and the display unit 16 may also serve as the operation unit 17.

### <Optical system>

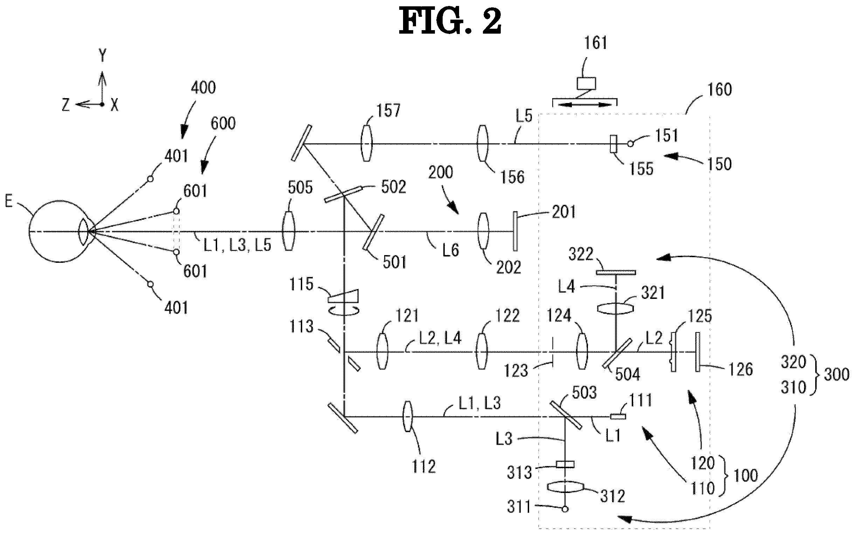

FIG. 2 is a schematic view of the optical system of the ophthalmic apparatus 10. As an example, the measurement unit 12 includes the first measurement optical system 100, the second measurement optical system 300, a fixation target presentation optical system 150, an observation optical system 200, an index projection optical system 400, an alignment index projection optical system 600, and the like. In addition, the measurement unit 12 also includes half mirrors 501 and 502, an objective lens 505, and the like for branching and coupling the optical paths of the respective optical systems.

### <First measurement optical system>

The first measurement optical system 100 is used to objectively measure the eye refractive power of the subject eye E. The first measurement optical system 100 has the projection optical system 110 and the light receiving optical system 120. The projection optical system 110 projects a spot-shaped measurement light flux on the fundus of the subject eye E through the center portion of the pupil of the subject eye E. The light receiving optical system 120 takes out the reflection light flux, reflected by the fundus, of the measurement light flux in a ring shape through the peripheral portion of the pupil.

The projection optical system 110 includes a light source 111, a relay lens 112, a hole mirror 113, a prism 115, an objective lens 505, and the like. The light source 111 is arranged on the optical axis L1 and arranged at the fundus conjugate position. The light source 111 may be a superluminescent diode (SLD) light source, a light emitting diode (LED) light source, or any other light source. In the present example, the light source 111 is an infrared light source, and an infrared light flux may be projected as the measurement light flux. For example, a near infrared light flux having a peak between wavelengths of 800 nm and 900 nm may also be used. As an example, a near infrared light flux having a peak wavelength of 870 nm may be used. The opening portion of the hole mirror 113 is arranged at the pupil conjugate position. The prism 115 is arranged at a position displaced from the pupil conjugate position, and makes the measurement light flux passing through the prism 115 with respect to the optical axis L1 eccentric. Instead of the prism 115, a plane-parallel plate may be arranged obliquely with respect to the optical axis L1.

The light receiving optical system 120 includes the objective lens 505, the prism 115, the hole mirror 113, relay lenses 121 and 122, a light receiving diaphragm 123, a collimator lens 124, a ring lens 125, the imaging element 126, and the like. The light receiving diaphragm 123 is arranged at the fundus conjugate position. The ring lens 125 is arranged at the pupil conjugate position. The imaging element 126 is arranged at the fundus conjugate position.

A measurement light flux from the light source 111 passes through the relay lens 112, the hole portion of the hole mirror 113, and the prism 115, is reflected by each of the half mirrors 502 and 501, and further reaches the fundus through the objective lens 505. The reflection light flux, reflected by the fundus, of the measurement light flux passes through the optical path through which the measurement light flux passed, is reflected by the mirror unit of the hole mirror 113, travels in the direction of the optical axis L2, and reaches the imaging element 126 via the relay lenses 121 and 122, the light receiving diaphragm 123, the collimator lens 124, and the ring lens 125.

In the present example, the prism 115 is arranged on the common optical path of the projection optical system 110 and the light receiving optical system 120. By rotating the prism 115 around the optical axis, the measurement light flux is eccentrically rotated at high speed on the pupil. As an example, in the present example, the measurement light flux is eccentrically rotated in a region of φ2 mm to φ4 mm on the pupil. This region becomes the eye refractive power measurement region (that is, the pupil region).

The control unit 80, which will be described later, analyzes the ring image formed on the imaging element 126 and derives the eye refractive power of the subject eye E. For example, an output image from the imaging element 126 or an added image of data sequentially output from the imaging element 126 is subjected to analysis processing to derive the eye refractive power. For example, each value of spherical power, cylindrical surface power, and astigmatism axis angle may be acquired.

The first measurement optical system 100 may be an optical system having a light projecting optical system that projects the measurement light flux onto the fundus of the subject eye E, and a light receiving optical system that receives the reflection light flux, reflected by the fundus, of the measurement light flux, and is not limited to the configuration described above. For example, the first measurement optical system 100 may be an optical system that projects a spot index onto the fundus and causes a Shack-Hartmann sensor to detect the reflection light flux of the spot index on the fundus. In this case, a Hartmann plate may be arranged instead of the ring lens 125. The reflection light flux of the spot index is separated into a plurality of light fluxes by the Hartmann plate, and the plurality of light fluxes are imaged by the imaging element 126 to detect a plurality of point images.

### <Second measurement optical system>

The second measurement optical system 300 is used to acquire contrast information of the subject eye E. The second measurement optical system 300 has the projection optical system 310 and the light receiving optical system 320. The projection optical system 310 projects a patterned measurement light flux onto the fundus of the subject eye E. The light receiving optical system 120 receives the reflection light flux, reflected from the fundus, of the measurement light flux.

The projection optical system 310 includes a light source 311, a lens 312, a pattern target plate 313, the relay lens 112, the hole mirror 113, the prism 115, the objective lens 505, and the like. The light source 311 is arranged on the optical axis L3 and arranged at the fundus conjugate position. The light source 311 may be any of an SLD light source, an LED light source, a light source different from SLD and LED, and the like. In the present example, the light source 311 is a visible light source, and a visible light flux may be projected as the measurement light flux. For example, a visible light flux having a peak between wavelengths of 500 nm and 580 nm may also be used. As an example, a visible light flux having a peak wavelength of 550 nm may be used. The pattern target plate 313 is arranged at the fundus conjugate position. The details of the pattern target plate 313 will be described later.

The light receiving optical system 320 includes the objective lens 505, the prism 115, the hole mirror 113, the relay lenses 121 and 122, the light receiving diaphragm 123, the collimator lens 124, a lens 321, the imaging element 322, and the like. The imaging element 322 is arranged at the fundus conjugate position.

In the present example, the light source 111 of the projection optical system 110 is arranged on the transmission side of the half mirror 503, and the light source 311, the lens 312, and the pattern target plate 313 of the projection optical system 310 are arranged on the reflection side of the half mirror 503. Accordingly, the optical axis L1 and the optical axis L3 are matched, and the respective optical paths are made common. Further, in the present example, the ring lens 125 and the imaging element 126 of the light receiving optical system 120 are arranged on the transmission side of a beam splitter 504, and the lens 321 and the imaging element 322 of the light receiving optical system 320 are arranged on the reflection side of the beam splitter 504. As a result, each optical path is branched, and the optical axis L2 and the optical axis L4 do not match.

Further, in the present example, the beam splitter 504 is arranged on the optical axis L2 (on the optical path) between the hole mirror 113 that branches the optical axis L1 (optical path) of the projection optical system 110 and the optical axis L2 (optical path) of the light receiving optical system 120, and the ring lens 125 included in the projection optical system 110.

The measurement light flux from the light source 311 passes through the lens 312 and the pattern target plate 313, is reflected by the half mirror 503, and reaches the fundus sequentially from the relay lens 112 to the objective lens 505. The reflection light flux, reflected by the fundus, of the measurement light flux is reflected by the mirror unit of the hole mirror 113 via the optical path through which the measurement light flux passed, sequentially passes from the relay lens 121 to the collimator lens 124, and is further reflected by the beam splitter 504, and reaches the imaging element 322 via the lens 321.

Further, in the present example, the measurement light flux from the light source 311 is eccentrically rotated by the prism 115, and contrast information is measured in a measurement region (pupil region) of φ2 mm to φ4 mm on the pupil.

The control unit 80 which will be described later acquires contrast information of the subject eye E by analyzing the pattern image formed on the imaging element 322. For example, an output image from the imaging element 126 or an added image of data sequentially output from the imaging element 126 is subjected to analysis processing to acquire the contrast information. For example, a contrast value may be obtained as the contrast information.

### <Image formation of first measurement optical system and second measurement optical system>

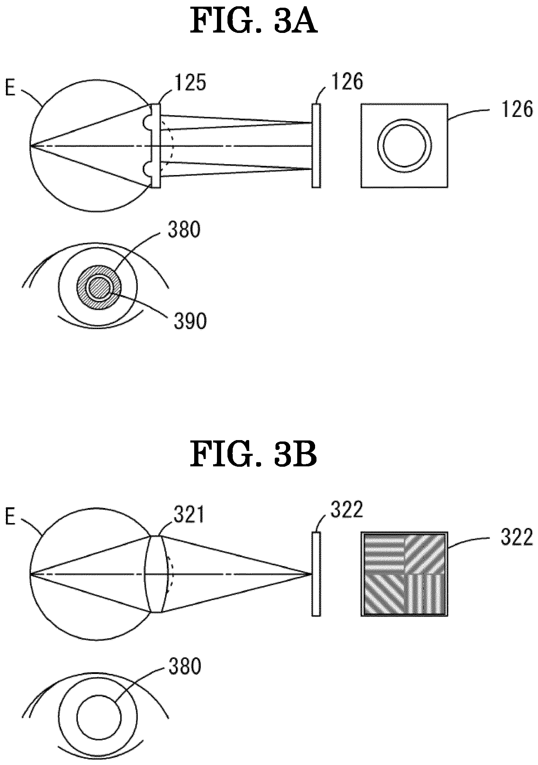

FIGs. 3A and 3B are schematic diagrams showing the reflection light flux from the fundus of the subject eye E and the pupil region. FIG. 3 A shows the case of the first measurement optical system 100. FIG. 3B shows the case of the second measurement optical system 300. Since the ring lens 125 of the first measurement optical system 100 and the lens 321 of the second measurement optical system 300 are arranged at the pupil conjugate positions, it is possible to consider that the ring lens 125 and the lens 321 are arranged on the pupil of the subject eye E. In the first measurement optical system 100, the reflection light flux from the fundus is directed toward a pupil region 380, but the light flux passing through a part of the pupil region 380 (that is, a pupil divided region 390 formed by dividing the pupil region 380) is taken out of the ring lens 125, and an image is formed on the imaging element 126 as a ring image. On the other hand, in the second measurement optical system 300, the reflection light flux from the fundus is directed toward the pupil region 380, the light flux passing through the entire pupil region 380 is taken out by the lens 321, and an image is formed as a pattern target image on the imaging element 322. For example, in this manner, in the second measurement optical system 300 with respect to the first measurement optical system 100, the entire pupil region 380 of the subject eye E (in other words, the effective diameter of the subject eye E) is used to acquire a pattern target image.

### <Fixation target presentation optical system>

The fixation target presentation optical system 150 presents a fixation target to the subject eye E. The fixation target presentation optical system 150 is used to fixate the subject eye E. In addition, the fixation target presentation optical system 150 is used to apply fog and adjustment load to the subject eye E.

The fixation target presentation optical system 150 includes a light source 151, a fixation target plate 155, lenses 156 and 157, the objective lens 505, and the like. The light source 151 is arranged on the optical axis L5. The fixation target plate 155 is arranged at the fundus conjugate position. A fixed light flux from the light source 151 passes through the fixation target plate 155, the lens 156, and the lens 157, passes through the half mirror 502, becomes coaxial with the optical axis L1, is reflected by the half mirror 501, and further reaches the fundus through the objective lens 505.

In the present example, the light source 111, the ring lens 125, and the imaging element 126 in the first measurement optical system 100, the light source 311, the lens 312, the pattern target plate 313, the lens 321, and the imaging element 322 in the second measurement optical system 300, and the light source 151 and the fixation target plate 155 in the fixation target presentation optical system 150 can be integrally moved along the optical axis as the driving unit 160. For example, by moving the driving unit 160 by the driving unit 161 according to the eye refractive power of the subject eye E, the presentation distance of the fixation target plate 155 with respect to the subject eye E (that is, the presentation position of the fixation target) can be changed. Similarly, by moving the driving unit 160, the presentation distance of the pattern target plate 313 with respect to the subject eye E (that is, the presentation position of the pattern target) can be changed.

In addition, the light source 111, the ring lens 125, and the imaging element 126 in the first measurement optical system 100, and the light source 311, the lens 312, the pattern target plate 313, the lens 321, and the imaging element 322 in the second measurement optical system 300, may be moved as one driving unit, and the light source 151 and the fixation target plate 155 in the fixation target presentation optical system 150 may be integrally moved as one driving unit. That is, the first measurement optical system 100, the second measurement optical system 300, and the fixation target presentation optical system 150 may be provided as independent driving units, respectively.

Furthermore, the light source 111 in the first measurement optical system 100 and the light source 311 to the pattern target plate 313 in the second measurement optical system 300 are used as one driving unit, and the ring lens 125 and the imaging element 126 in the first measurement optical system 100 and the lens 321 and the imaging element 322 in the second measurement optical system 300 may be used as one driving unit. That is, each of the projection optical systems of the first measurement optical system 100 and the second measurement optical system 300, each of the light receiving optical systems of the first measurement optical system 100 and the second measurement optical system 300, and the fixation target presentation optical system 150 may be provided as independent driving units, respectively.

### <Observation optical system>

The observation optical system 200 is used to capture an observation image of the anterior eye part of the subject eye E. The observation image is used for alignment and the like. For example, the observation optical system 200 includes an imaging element 201, a lens 202, the objective lens 505, and the like. An imaging element 205 is arranged at the pupil conjugate position. The observation optical system 200 also serves as a detection optical system for detecting the index image projected onto the cornea from the index projection optical system 400 and the index image projected onto the cornea from the alignment index projection optical system 600.

### <Index projection optical system>

The index projection optical system 400 is used to measure the corneal shape. The index projection optical system 400 projects an index for measuring the corneal shape from the front facing the subject eye to the anterior eye part. The index projection optical system 400 has a plurality of point light sources 401. The point light source 401 projects an infinite distance index by irradiating the cornea with a parallel light flux. The point light sources 401 are arranged vertically and horizontally symmetrically about the optical axis L1. For example, in the present example, two point light sources are provided respectively on the right and left sides. Accordingly, four point image indices are projected onto the cornea. Note that the number of indices is not limited to this, and may be composed of three or more point image indices. Further, the shape of the index is not limited to this, and a linear index or the like may be included.

### <Alignment index projection optical system>

The alignment index projection optical system 600 is used to align the measurement unit 12 with the subject eye E. The alignment index projection optical system 600 includes an alignment light source 601. The alignment light source 601 projects a finite distance index by irradiating the cornea with a diffused light flux. The alignment light source 601 is arranged in a ring shape around the optical axis L1. Thereby, a ring index (so-called mire ring image) is projected onto the cornea.

In the present example, the alignment light source 601 and the index projection optical system 400 form an alignment index projection optical system. For example, the working distance may be adjusted by moving the measurement unit 12 in the front-rear direction such that the Purkinje image by the alignment light source 601 and the Purkinje image by the index projection optical system 400 are captured at a predetermined ratio.

### <Pattern target plate>

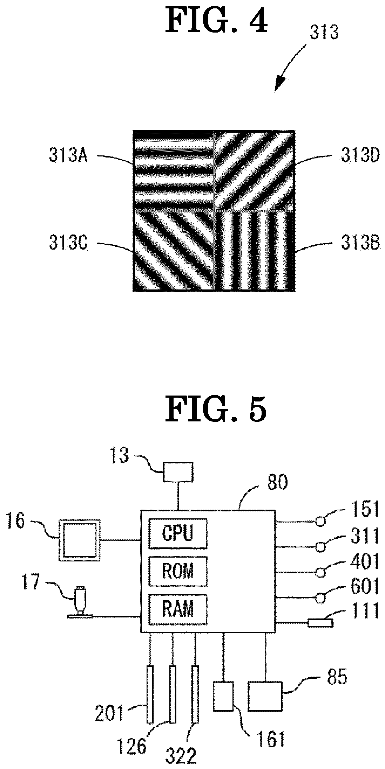

FIG. 4 shows an example of the pattern target plate 313. The pattern target plate 313 is composed of striped patterns of which luminance changes along at least one direction. For example, a striped pattern in which the luminance alternately increases and decreases may be used. In addition, for example, a striped pattern using a so-called sine wave grating, in which the luminance changes according to a sine wave function, may be used. In this case, the higher the frequency in the sine wave function, the narrower the fringe interval, and the lower the frequency, the wider the fringe interval. However, the frequency may be preset as a fixed value, or may be changed to any value.

In the present example, the pattern target plate 313 has patterns in four directions including a pattern 313A that changes periodically in the vertical direction (90-degree direction), a pattern 313B that changes periodically in the horizontal direction (180 -degree direction), and patterns 313C and 313D that change periodically in the diagonal direction (45 degrees and 135 degrees). Note that the pattern target plate 313 may have a pattern in at least one of these four directions. That is, only the pattern 313A may be provided. Alternatively, only the pattern 313B may be provided. Alternatively, only the pattern 313C may be provided. Alternatively, only the pattern 313D may be provided. In addition, a combination of a plurality of patterns may be used. It is needless to say that the pattern target plate 313 may have a striped pattern such as the patterns 313A to 313D, as well as Landolt rings, letters, numbers, and the like.

### <Control system>

FIG. 5 is a schematic view of the control system of the ophthalmic apparatus 10. The control unit 80 includes a CPU (processor), a RAM, a ROM, and the like. The CPU controls driving of each unit in the ophthalmic apparatus 10. The RAM temporarily stores various information. Various programs and the like executed by the CPU are stored in the ROM. The control unit may be configured with a plurality of control units (that is, a plurality of processors).

The control unit 80 is electrically connected to the alignment driving unit 13, the display unit 16, the operation unit 17, the non-volatile memory 85 (hereinafter referred to as the memory 85), and the like. In addition, each light source, each imaging element, each driving unit, and the like of the measurement unit 12 are electrically connected to the control unit 80. The memory 85 is a non-transitory storage medium that can hold stored contents even when power supply is interrupted. For example, as the memory 85, a hard disk drive, a flash ROM, a USB memory, or the like can be used. The memory 85 may store the measurement results of the subject eye E and the like.

### <Control operation>

A control operation of the ophthalmic apparatus 10 will be described. In the present example, the ophthalmic apparatus 10 sequentially acquires the eye refractive power and contrast information of the subject eye E.

### <Alignment>

The examiner guides the examinee in front of the ophthalmic apparatus 10 and instructs the subject to put the face on the face support unit 15, and selects a button for starting measurement of the eye refractive power of the subject eye E. The control unit 80 aligns the measurement unit 12 with respect to the subject eye E based on a control signal from the operation unit 17.

For example, the control unit 80 turns on the light source 151 of the fixation target presentation optical system 150 to present the fixation target to the subject eye E. Further, each light source of the index projection optical system 400 and the alignment index projection optical system 600 is turned on, and the alignment index is projected onto the cornea of the subject eye E. For example, the control unit 80 detects an alignment index image (a point image and a mire ring image) from an observation image captured by the imaging element 201 of the observation optical system 200, and obtains the deviation in the X direction, the Y direction, and the Z direction of the measurement unit 12 with respect to the subject eye E based on the alignment index image, and the measurement unit 12 is moved. Accordingly, the alignment is completed.

### <Acquisition of eye refractive power>

When the alignment of the subject eye E and the measurement unit 12 is completed, the eye refractive power of the subject eye E is measured. For example, in the measurement of the eye refractive power, preliminary measurement may be performed first, and main measurement may be performed later.

In the preliminary measurement of the subject eye E, the eye refractive power is measured in a state where the fixation target is arranged at a predetermined presentation distance. For example, the control unit 80 arranges the fixation target plate 155 at an initial position that is optically sufficiently far away from the subject eye E and corresponds to the far point of the 0D eye. In addition, the light source 111 of the projection optical system 110 emits the measurement light flux, and the ring image captured by the imaging element 126 of the light receiving optical system 120 is subjected to image analysis. For example, the control unit 80 thins the ring image to obtain the eye refractive power in each meridian direction, performs predetermined processing on the eye refractive power, and acquires at least the spherical power (the spherical power in the preliminary measurement).

Subsequently, fog is added to the subject eye E. For example, the control unit 80 arranges the fixation target plate 155 at the fog start position where the subject eye E is in focus, according to the spherical power of the preliminary measurement of the subject eye E. The subject eye E becomes able to clearly observe the fixation target. After that, the control unit 80 moves the fixation target from the fog start position. The subject eye E is no longer focused on the fixation target, and fog is added. As a result, the adjustment of the subject eye E is released, and the eye refractive power approaches the true value.

In the main measurement of the subject eye E, the eye refractive power is measured in a state where fog is added to the subject eye E. For example, the control unit 80 causes the imaging element 226 to continuously capture ring images at each predetermined timing. Further, for example, the control unit 80 adds the ring image to reduce noise light, acquires the eye refractive power (the spherical power, the cylindrical power, and the astigmatism axis angle in the main measurement) by the same method as the preliminary measurement, stores the eye refractive power in the memory 75.

Here, the subject eye E may be an intraocular lens implanted eye or an eye without an intraocular lens. When the subject eye E is the intraocular lens implanted eye, depending on the type of intraocular lens (for example, monofocal intraocular lens, EDoF type intraocular lens, and multifocal intraocular lens), there is a possibility that the eye refractive power cannot be measured correctly. For example, in the EDoF type intraocular lens and the multifocal intraocular lens, measurement results are likely to vary depending on whether the lenses are refractive or diffractive. However, the examiner does not necessarily understand whether the subject eye E is an intraocular lens implanted eye. Further, even when the examiner understands that the subject eye E is an intraocular lens implanted eye, the examiner may not know the type of the intraocular lens. Therefore, it is difficult for the examiner to determine whether the measurement result of the eye refractive power of the subject eye E is appropriate.

In the present example, by acquiring the contrast information (here, contrast value) of the subject eye E, it is possible to distinguish whether the subject eye E is an intraocular lens implanted eye. In addition, it is possible to estimate the type of intraocular lens. Further, it is possible to make appropriate prescriptions for the intraocular lens implanted eye.

### <Acquisition of contrast information>

When the eye refractive power of the subject eye E is acquired, the contrast value of the subject eye E is measured. For example, a plurality of contrast values with different diopter information may be measured by changing the diopter information of the subject eye E (for example, information on at least one of the spherical power, the cylindrical power, and the astigmatism axis angle). Here, a case will be exemplified in which the spherical power of the subject eye E is continuously changed and the accompanying continuous change in the contrast value is measured.

The contrast value of the subject eye E is measured based on the eye refractive power of the subject eye E in a state where the pattern target plate 313 is arranged at a predetermined presentation distance. For example, the distance at which the subject eye E has a spherical power of 0D may be set as the predetermined presentation distance of the pattern target plate 313. The control unit 80 drives the driving unit 161 to move the driving unit 160, thereby arranging the pattern target plate 313 at the position D1 corresponding to 0D (that is, the measurement start position D1 of the contrast value). Subsequently, the control unit 80 changes the spherical power added to the subject eye E. For example, by gradually moving the pattern target plate 313 from the measurement start position D1, the spherical power is changed from 0D to a negative value. Further, when the pattern target plate 313 reaches a predetermined presentation distance (that is, the measurement end position D2 of the contrast value), the control unit 80 stops the movement of the pattern target plate 313. Note that the measurement end position D2 may be preset or may be set in any manner. At this time, since the fixation target plate 155 moves at the same time as the pattern target plate 313, the fixation target is also initially arranged at a position corresponding to 0D, and the subject eye E is focused on the fixation target. As the fixation target moves away from the position corresponding to 0D, the fixation target is gradually out of focus.

While the pattern target plate 313 is moving from the measurement start position D1 to the measurement end position D2, the control unit 80 causes the light source 311 of the projection optical system 310 to emit the measurement light flux, and the imaging element 322 of the light receiving optical system 320 to capture the pattern target image. For example, a pattern target image may be captured each time the spherical power changes by one step (for example, 0.25 D). It is needless to say that the interval of one step is not limited to this, and may be an interval smaller than 0.25 D or may be an interval larger than 0.25 D. The pattern target image is stored in the memory 85 in association with the position of the pattern target plate 313 (that is, the presentation distance of the pattern target).

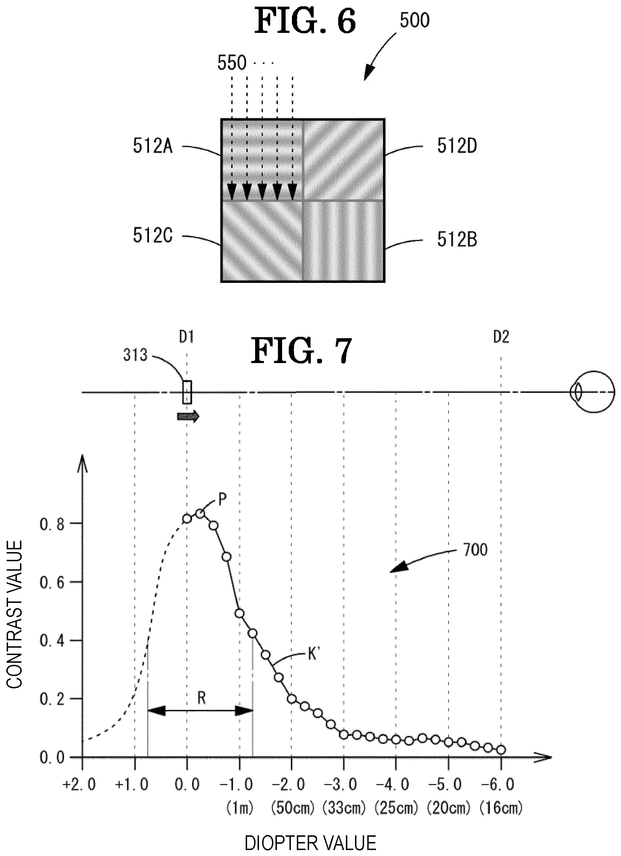

FIG. 6 is an example of a pattern target image 500. The pattern target image 500 includes a pattern image 512A that is an image of the pattern 313A, a pattern image 512B that is an image of the pattern 313B, a pattern image 512C that is an image of the pattern 313C, and a pattern image 512D that is an image of the pattern 313D, and is captured by the imaging element 322 as a single image including each of the pattern images. The control unit 80 thins the pattern target image 500 and acquires luminance information along the direction in which each pattern changes periodically. For example, luminance information in the vertical direction is acquired for the pattern image 512A that changes periodically in the vertical direction. As an example, the maximum value Imax and the minimum value Imin of luminance in at least one pixel line 550 in the vertical direction are calculated, and the contrast value Ka in the vertical direction is measured by obtaining contrast value K = (Imax - Imin) / (Imax + Imin). The width of the pixel line 550 may be 1 pixel width or 2 pixel width or more. Further, the number of pixel lines 550 may be two or more, and when a plurality of lines are provided, the average thereof may be used as the contrast value.

Similarly, for the pattern image 512B that changes periodically in the horizontal direction, the control unit 80 also calculates the maximum value Imax and the minimum value Imin of the luminance as luminance information in the horizontal direction, and measures the contrast value Kb in the horizontal direction. In addition, the contrast value Kc in the diagonal direction is measured for the pattern image 512C that changes periodically in the diagonal direction. Further, the contrast value Kd in the diagonal direction is measured for the pattern image 512D that changes periodically in the diagonal direction.

Furthermore, the control unit 80 averages the contrast values Ka to Kd in the vertical direction, the horizontal direction, and the diagonal direction to calculate the overall contrast value K' of the pattern target image 500 at the position of the pattern target plate 313. The control unit 80 may calculate the overall contrast value K' for each of the pattern target images 500 associated with the positions of the pattern target plate 313. Thereby, the contrast value K' corresponding to the position of the pattern target plate 313 (that is, the contrast value K' corresponding to the spherical power of the subject eye E) can be measured.

### <Output of change information>

Both the measurement results of the eye refractive power and the contrast information of the subject eye E are displayed on the display unit 16. As described above, the measurement result of the eye refractive power of the subject eye E may not be appropriate, but may be displayed as a reference value. Further, the contrast information of the subject eye E may be displayed as contrast value change information with respect to the diopter information of the subject eye E.

FIG. 7 is an example of contrast value change information with respect to diopter information of the subject eye E. In the present example, a graph 700 showing the distribution of the contrast value K' with respect to the diopter information (spherical power) of the subject eye E is output. Since the change in the spherical power of the subject eye E corresponds to the change in the position of the pattern target plate 313, the change in the spherical power can also be expressed as a graph showing the distribution of the contrast value K' with respect to the position of the pattern target plate 313. On the graph 700, a plurality of contrast values K' with different spherical powers of the subject eye E are plotted. For example, at least the data of the peak value P of the contrast value K' and the peripheral value of the peak value P may be plotted. It should be noted that, for the peripheral value of the peak value P, a predetermined range of spherical powers may be preset, or the peripheral value may be changed to any range. For example, the peripheral value may be a value within the absolute value range of 1.0 (range R in FIG. 7) with the peak value P as a reference.

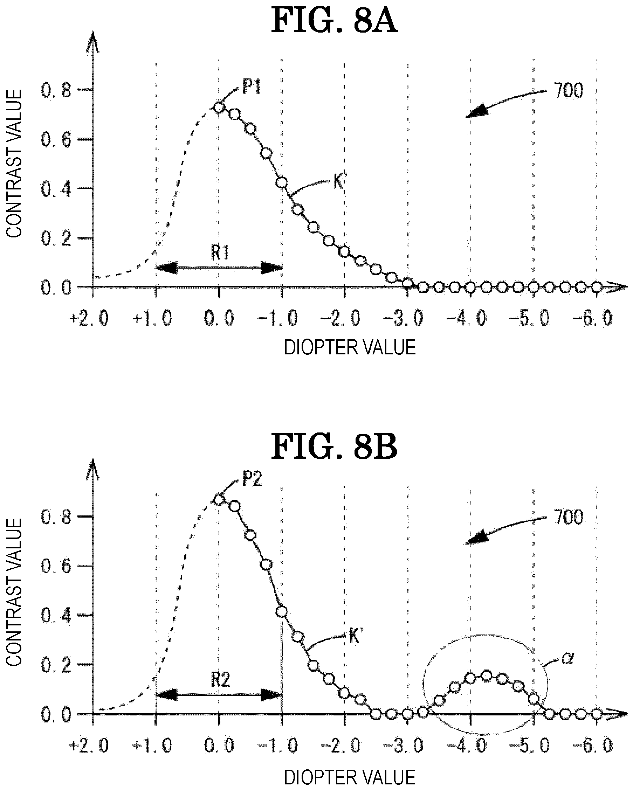

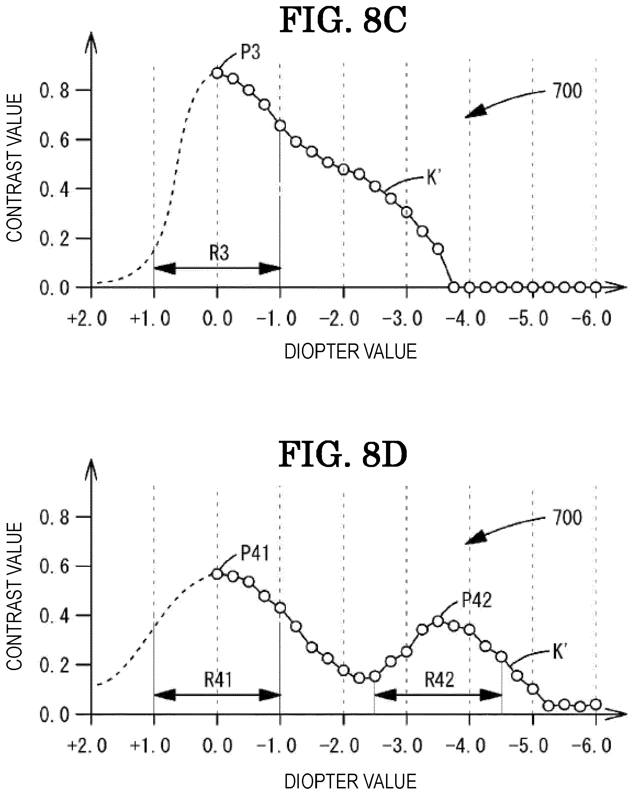

FIGs. 8A to 8D are examples of a graph corresponding to the subject eye E. FIG. 8A shows a case where the subject eye E is an eye without an intraocular lens. FIG. 8B shows a case where the subject eye E is a monofocal intraocular lens implanted eye. FIG. 8C shows a case where the subject eye E is an EDoF intraocular lens implanted eye. FIG. 8D shows a case where the subject eye E is a multifocal intraocular lens implanted eye. The horizontal axis is the spherical power of the subject eye E, and the vertical axis is the contrast value K'.

When the subject eye E is an eye without an intraocular lens, the graph 700 plots data including one peak value P1 and at least a part of the range R1 with the peak value P1 as a reference. Further, when the subject eye E is a monofocal intraocular lens implanted eye, the graph 700 plots data including one peak value P2 and at least a part of the range R2 with the peak value P2 as a reference. In addition, when the subject eye E is an EDoF intraocular lens implanted eye, the graph 700 plots data including one peak value P3 and at least a part of the range R3 with the peak value P3 as a reference. Further, when the subject eye E is a multifocal intraocular lens implanted eye, the graph 700 plots data including a plurality of peak values and a spherical power range with each peak value as a reference. For example, data including two peak values P41 and P42 and spherical power ranges R41 and R42 are plotted.

When the examiner confirms the single peak from the graph 700, the examiner can easily determine whether the subject eye E is the eye without an intraocular lens, the monofocal intraocular lens implanted eye, or the EDoF intraocular lens implanted eye. It should be noted that the eye without an intraocular lens, the monofocal intraocular lens implanted eye, and the EDoF intraocular lens implanted eye can be determined from the width of a single peak. For example, the width of the single peak is wider in the EDoF intraocular lens implanted eye than in the eye without an intraocular lens and the monofocal intraocular lens implanted eye. Further, when the examiner confirms the double peak from the graph 700, the examiner E can easily determine that the subject eye E is the eye in which a multifocal intraocular lens (bifocal intraocular lens) is implanted. Furthermore, when the type of intraocular lens is a multifocal type, the distribution of the contrast value K' can be used to understand the near focal position relative to the far focal position, the distribution of light between the distant and near areas, and the like.

It is also possible for the examiner to make prescriptions for the subject eye in consideration of the information obtained from the contrast information. As an example, when a monofocal intraocular lens or an EDoF intraocular lens is inserted into the subject eye and it is desired to prescribe spectacles to compensate for near vision, the dioptric power of the lens can be determined in consideration of these information. In addition, for example, when it is necessary to add a new intraocular lens to the subject eye or to change the intraocular lens, the dioptric power of the lens can be selected in consideration of visual performance with current intraocular lenses. For example, by showing the characteristics of the intraocular lens from the change information of the contrast value K' with respect to the spherical power of the subject eye E, it becomes easier to imagine the necessary prescriptions for the subject eye.

When the subject eye E is the eye in which a monofocal intraocular lens, an EDoF intraocular lens, or a multifocal intraocular lens is implanted, the graph 700 may have a peak α corresponding to spurious resolution. For example, although the contrast value K' became zero once as the spherical power of the subject eye E changed, the peak that appears when the contrast value K' increases again is the peak α corresponding to spurious resolution. The examiner can determine the presence or absence of spurious resolution by observing the change in the contrast value K'.

The spurious resolution of the contrast value K' can also be distinguished at the stage of image processing of the pattern target image 500. For example, the arrangement of the black and white striped patterns on the pattern target plate 313 and the arrangement of the black and white striped patterns on the pattern target image 500 usually match each other. However, in the case of spurious resolution, the arrangement of white and black striped patterns on the pattern target image 500 is reversed with respect to the arrangement of white and black striped patterns on the pattern target plate 313. Therefore, the control unit 80 may treat the spurious resolution part of the contrast value K' as zero and display this on the graph 700. In addition, the control unit 80 may notify that the contrast value K' includes spurious resolution by changing the color of the broken line, displaying a message, or the like.

In the present example, the pattern target image 500 may be displayed together with the graph 700 described above. For example, the pattern target image 500 may be displayed for each predetermined spherical power (for example, for each change of 1.0D). In addition, for example, at least the pattern target image 500 corresponding to the peak value P may be displayed. Further, for example, at least the pattern target image 500 corresponding to the peak value P and the peripheral value of the peak value P may be displayed. In the second measurement optical system 300, the reflection light flux from the fundus passes through the entire pupil region (the effective diameter of the subject eye E) and is imaged by the imaging element 322. Therefore, the pattern target image 500 can be considered as the actual visual performance of the pattern target plate 313 (the visual performance in a state where the distant image and the near image are superimposed). Therefore, by displaying the pattern target image 500, the visual performance of the subject eye E can be intuitively understood.

Further, in the present example, the clear vision region of the subject eye E may be displayed together with the graph 700 described above. The clear vision region of the subject eye E may be a region in which the subject eye E can focus. For example, the clear vision region may be indicated by obtaining at least the far point (for example, the peak value P) from the distribution of the contrast values K' with respect to the spherical power of the subject eye E. The width of the clear vision region may be preset as a fixed value, or may be changed to any value.

It is needless to say that the clear vision region of the subject eye E may be indicated by a method different from the above. For example, a range in which the contrast value K' is a predetermined percentage or more with respect to the peak value P of the contrast value K' with respect to the spherical power of the subject eye E may be indicated as the clear vision region. Further, for example, a range in which the contrast value K' with respect to the spherical power of the subject eye E is equal to or greater than a predetermined threshold value may be indicated as the clear vision region. Further, for example, the clear vision region may be set based on the subjective eye refractive power and visual acuity value of the subject eye E.

It is needless to say that both the pattern target image 500 and the clear vision region of the subject eye E may be displayed together with the graph 700 described above.

Further, in the present example, a graph 700 showing the contrast value K' with respect to the spherical power of one eye of the subject eye E may be displayed. Further, a graph 700 showing the contrast value K' with respect to the spherical power of both eyes of the subject eye E may be displayed. In this case, two curves may be displayed by superimposing the contrast value K' for the left eye spherical power and the contrast value K' for the right eye spherical power, or one curve obtained by synthesizing the contrast value K' for the left eye spherical power and the contrast value K' for the right eye spherical power may be displayed. Note that at least one of addition processing, averaging processing, weighting processing, and the like may be executed when synthesizing each of the contrast values K'. Accordingly, the binocular vision function of the subject eye E can be taken into consideration.

As described above, for example, the ophthalmic apparatus of the present example acquires the optical characteristics of the subject eye, acquires at least a plurality of contrast information with different diopter information in the subject eye, and outputs the change information of the contrast information with respect to the diopter information. Accordingly, it becomes easier to understand whether the subject eye is the intraocular lens implanted eye. In addition, when the subject eye is the intraocular lens implanted eye, it becomes easier to understand what kind of characteristics the lens is inserted. In addition, it is possible to determine whether the measurement result of the optical characteristics of the subject eye (for example, the eye refractive power) is appropriate by using the contrast information. Furthermore, the characteristics of the intraocular lens and the like can be acquired from the change information, and it becomes easier to derive appropriate prescriptions necessary for the subject eye.

In addition, for example, the ophthalmic apparatus of the present example includes, as change information, first data indicating a peak value of contrast information with respect to diopter information. Accordingly, for example, the positions of the far point and the near point of the subject eye can be understood.

In addition, for example, the ophthalmic apparatus of the present example includes, as change information, second data indicating peripheral value for the first data of the contrast information with respect to the diopter information. Accordingly, it is possible to predict how the characteristics of the intraocular lens change before and after the far point and near point of the subject eye. In addition, by being able to predict the characteristics of the intraocular lens, it is possible to estimate the type of the intraocular lens.

Further, for example, the ophthalmic apparatus of the present example outputs, as change information, a graph showing the distribution of contrast information with respect to diopter information. As a result, the relationship between the diopter information and the contrast information can be expressed visually in an easy-to-understand manner, and it becomes easier to predict the characteristics of the intraocular lens and estimate the type of the intraocular lens.

In addition, for example, the ophthalmic apparatus of the present example outputs a graph continuously showing a change in contrast information accompanied by a change in diopter information. Accordingly, it is possible to easily understand the positions of the far point and the near point of the subject eye, changes in the characteristics of the intraocular lens before and after the far point and the near point, and the like. Further, the type of intraocular lens inserted into the subject eye can be easily determined. In addition, when spectacle prescriptions or the like are required for the subject eye E, the calibration amount can be determined in consideration of such information. Further, for example, when the subject eye needs to newly add an intraocular lens or change the intraocular lens, it is possible to select an appropriate lens.

Further, for example, the ophthalmic apparatus of the present example acquires contrast information with different diopter information of the subject eye based on the optical characteristics of the subject eye. For example, contrast information with different diopter information may be acquired based on the eye refractive power of the subject eye. In this case, the position of the optical characteristics (eye refractive power) of the subject eye or the position around the optical characteristic (eye refractive power) of the subject eye can be considered as the position of the far point of the subject eye. As a result, changes in the characteristics of the intraocular lens inserted into the subject eye can be easily captured, and prescriptions required for the subject eye can be easily determined.

Further, for example, in the ophthalmic apparatus of the present example, the second measurement light flux projected to acquire the contrast information of the subject eye is a patterned light flux, a periodically changing index pattern image is detected by the second reflection light flux formed by the second measurement light flux from the fundus, and the contrast information is acquired based on the luminance information in this index pattern image. For example, contrast information of a subject eye can be easily acquired by using a patterned light flux that changes periodically. Further, it is possible to acquire optical characteristics based on the contrast information of the subject eye. In addition, it is also possible to calculate the astigmatism information (for example, cylindrical power and astigmatism axis angle) of the subject eye by using the patterned light flux that changes periodically in the plurality of different directions.

Further, for example, the ophthalmic apparatus of the present example includes: an eye refractive power measuring optical system including a first light projecting optical system that projects a first measurement light flux toward a fundus of the subject eye, and a first light receiving optical system in which a first detector detects a first reflection light flux, reflected by the fundus, of the first measurement light flux as a first index pattern image, and for acquiring an eye refractive power of the subject eye; and a contrast measuring optical system including a second light projecting optical system that projects a patterned second measurement light flux toward the fundus of the subject eye, and a second light receiving optical system in which a second detector detects a second index pattern image formed by a second reflection light flux, reflected by the fundus, of the second measurement light flux, and for acquiring contrast information of the subject eye. The first detector and the second detector are different detectors. Accordingly, eye refractive power and contrast information can be acquired without complicating each optical system.

For example, in the ophthalmic apparatus of the present example, in the first light receiving optical system, the first detector detects the first reflection light flux, which has passed through a pupil divided region formed by dividing a pupil region of the subject eye, as the first index pattern image, and in the second light receiving optical system, the second detector detects the second index pattern image formed by the second reflection light flux which has passed through the pupil region of the subject eye. Since the second detector detects the reflection light flux that has passed through the effective diameter of the subject eye in the second light receiving optical system, it is possible to acquire contrast information that reflects the actual visual performance of the subject eye.

In addition, for example, in the ophthalmic apparatus of the present example, a first optical path branching member branches an optical path of the first light projecting optical system and an optical path of the first light receiving optical system, a second optical path branching member branches an optical path of the first light receiving optical system and an optical path of the second light receiving optical system, and the second optical path branching member is arranged between the first optical path branching member and the pattern optical member to dispose the first detector and the second detector in different optical paths. For example, when the first detector and the second detector are used in common and arranged on the same optical path, as the second reflection light flux passes through the pattern optical member, the second index pattern image is divided into a plurality of images, and it becomes difficult to acquire contrast information. However, by branching the optical path before the second reflection light flux passes through the pattern optical member, the second index pattern image can be detected as one image, and the contrast information can be acquired appropriately.

Further, for example, in the ophthalmic apparatus of the present example, the first measurement light flux for measuring the eye refractive power of the subject eye is an infrared light flux, and the second measurement light flux for measuring the contrast information of the subject eye is visible light flux. As a result, the examinee is less likely to feel glare when the first measurement light flux is projected, miosis is suppressed, and the eye refractive power can be obtained with high accuracy. In addition, accordingly, regardless of whether the subject eye is the intraocular lens implanted eye, contrast information can be acquired satisfactorily. When the second measurement light flux is an infrared light flux, there is a possibility that the contrast information cannot be measured correctly depending on the type of intraocular lens (in particular, a multifocal intraocular lens), but when the second measurement light flux is a visible light flux, it is possible to respond to various intraocular lenses, and the contrast information can be obtained with high accuracy.

### <Modification example>

In the present example, a configuration is described as an example in which the light source 111 emits an infrared light flux as a measurement light flux in the projection optical system 110 of the first measurement optical system 100, but the present invention is not limited thereto. For example, an optical member for limiting a specific wavelength may be arranged in the optical path of the measurement light flux from the light source 111. Similarly, in the present example, a configuration is described as an example in which the light source 311 emits a visible light flux as a measurement light flux in the projection optical system 310 of the second measurement optical system 300, but the present invention is not limited thereto. For example, an optical member for limiting a specific wavelength may be arranged in the optical path of the measurement light flux from the light source 311. For example, the optical member that limits the wavelength of each measurement light flux may be a cut filter or the like.

In the present example, a configuration is described as an example in which the measurement light flux from the projection optical system 310 is the visible light flux, but the present invention is not limited thereto. The measurement light flux from the projection optical system 310 may be an infrared light flux. In this case, the subject eye E is less likely to feel glare during the measurement of the contrast value, and the load is reduced. On the other hand, when the subject eye E is the multifocal intraocular lens implanted eye, there is a possibility that measurement results cannot be acquired with an infrared light flux, especially with a diffraction type that divides the visible light flux into far and near areas based on the principle of diffraction. Therefore, the ophthalmic apparatus 10 may be provided with a visible light source and an infrared light source as the light source 311 of the projection optical system 310, and switch these light sources as necessary to obtain the contrast value K'. For example, when measurement results using an infrared light source are not obtained, measurement results may be obtained by switching to a visible light source.

In the present example, a configuration is described as an example in which both a fixation target and a pattern target are presented to the subject eye E in acquiring the contrast information of the subject eye E, but the present invention is not limited thereto. In the present example, the measurement light flux from the projection optical system 310 and the fixed light flux from the fixation target presentation optical system 150 are both visible light fluxes, and thus the fixation target and the pattern target may be superimposed on the subject eye E. Therefore, only the pattern target may be presented at the time of acquiring the contrast information of the subject eye E, and the pattern target may also serve as the fixation target.

In addition, when acquiring the contrast information of the subject eye E, the measurement light flux from the projection optical system 310 is a visible light flux, and accordingly, the subject eye E may feel glare during the measurement of the contrast value, and miosis may be caused. Therefore, the amount of light from the light source 311 that illuminates the pattern target plate 313 may be adjusted such that the pattern target is presented to the subject eye E with a brightness that is approximately the same as or lower than the brightness of the fixation target. Further, the exposure time and gain of the imaging element 322 may be adjusted such that the pattern target image can be easily detected.

In the present example, a configuration is described as an example in which the pattern target plate 313 is moved with respect to the subject eye E and the fixation target plate 155 is moved in acquiring the contrast information of the subject eye E, but the present invention is not limited thereto. For example, when the subject eye E is the intraocular lens implanted eye, there is a possibility that the fixation target plate 155 will move to cause adjustment. Therefore, the fixation target plate 155 may be fixedly arranged.

In the present example, a configuration is described as an example in which the contrast value K' is measured in a state where the pattern target plate 313 arranged at the measurement start position D1 where the spherical power of the subject eye E is 0D is arranged based on the measurement result of the eye refractive power of the subject eye E, but the present invention is not limited thereto. When the eye refractive power of the subject eye E is not a correct measurement result, the peak value P of the contrast value K' necessarily comes at a distance where the spherical power of the subject eye E is 0D (or a distance closer than 0D).

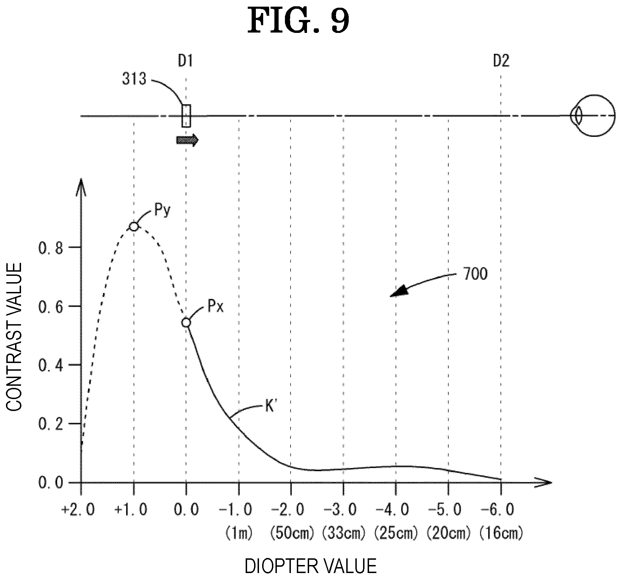

FIG. 9 is an example in which a peak value appears at a distance different from the spherical power of 0D. For example, when the pattern target plate 313 is moved from the measurement start position D1 (the distance at which the spherical power is 0D) and such a graph 700 is obtained, the contrast value K' at the measurement start position D1 becomes the peak value Px. However, it is unknown how the contrast value K' changes on the far side of the measurement start position D1, and in fact, the true peak value Py may appear at a distance of spherical power +1.0D. Therefore, a predetermined distance at which the spherical power of the subject eye E is a positive value (for example, spherical power +2.0D) may be preset as the measurement start position D1. In addition, the measurement start position D1 may be set in any manner. As a result of moving the pattern target plate 313 toward the near side from the distance with the spherical power of 0D, when the distance with the spherical power of 0D reaches the peak value, the pattern target plate 313 is further moved from the distance with the spherical power of 0D to the far side to detect whether there is a true peak value.

In the present example, a configuration is described as an example in which, as the diopter information of the subject eye E, a graph 700 with the horizontal axis representing the spherical power is output, but the present invention is not limited thereto. The diopter information of the subject eye E may be the cylindrical power or the astigmatism axis angle. In this case, the second measurement optical system 300 may have a configuration for changing the cylindrical power of the subject eye E, or may have a configuration for changing the astigmatism axis angle of the subject eye E. For example, two rotatable cylindrical lenses may be provided.

In the present example, a configuration is described as an example in which the graph 700 obtained by plotting the overall contrast value K' obtained by averaging the contrast values Ka to Kd in each direction included in the pattern target image 500 is output, but the present invention is not limited thereto. For example, a graph 700 obtained by separately plotting contrast values in at least two directions may be output. As an example, the contrast value Ka in the vertical direction and the contrast value Kb in the horizontal direction may be plotted separately. In this case, the contrast value Ka and the contrast value Kb may be displayed in one graph, or may be displayed as separate graphs.

In the present example, a configuration is described as an example in which a graph 700 showing the relationship between the spherical power of the subject eye E and the contrast value K' is output, but the present invention is not limited thereto. When the pattern target image 500 includes pattern images in at least three directions, a graph showing the relationship between the spherical power of the subject eye E and the peak value P of the contrast value in each direction may be output. Accordingly, the eye refractive power based on the contrast value of the subject eye E can be calculated.

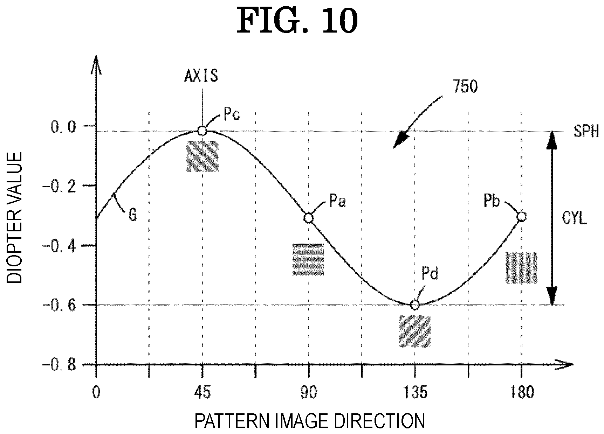

FIG. 10 is an example of a graph 750 showing a relationship between the spherical power of the subject eye E and the peak value P of the contrast value in each direction. The horizontal axis is the direction (angle) of the pattern image, and the vertical axis is the spherical power of the subject eye E. For example, by plotting each of a peak value Pa of the contrast value in the pattern image 512A in the horizontal direction (90 -degree direction), a peak value Pb of the contrast value in the pattern image 512B in the vertical direction (180 - degree direction), a peak value Pc of the contrast value in the pattern image 512C in the diagonal direction (45 degrees direction), and a peak value Pd of the contrast value in the pattern image 512D in the diagonal direction (135-degree direction), a dioptric power distribution curve G passing through each peak value is obtained. For example, in the dioptric power distribution curve G, the control unit 80 may set the maximum value to the spherical power SPH, the difference between the maximum value and the minimum value to the cylindrical power CYL, and the direction (angle) of the maximum value to the astigmatism axis angle AXIS to calculate the eye refractive power of the subject eye E.

In the above configuration, the first eye refractive power (the first spherical power, the first cylindrical power, and the first astigmatism axis angle) based on the eye refractive power measurement of the subject eye E, and the second eye refractive power (the second spherical power, the second cylindrical power, and the second astigmatism axis angle) based on the contrast measurement of the subject eye E are acquired, and thus the first eye refractive power may be corrected based on the second eye refractive power. For example, in an intraocular lens implanted eye, in eye refractive power measurement using the first measurement optical system 100, the spherical power of the subject eye E may be calculated as a deviated value. Moreover, even though the subject eye E has no astigmatism, the cylindrical power and the astigmatism axis angle may be calculated. Therefore, the control unit 80 obtains the deviation amount between the first eye refractive power and the second eye refractive power, and adds (or subtracts) the deviation amount to (or from) the first eye refractive power, and accordingly, the value of the first eye refractive power may be corrected. For example, the value of the first spherical power may be corrected to the value of the second spherical power. Further, for example, the value of the first cylindrical power may be corrected to the value of the second cylindrical power. Further, for example, the value of the first astigmatism axis angle may be corrected to the value of the second astigmatism axis angle. In addition, a predetermined allowable range may be provided for the deviation amount between the first eye refractive power and the second eye refractive power, and correction may be executed when the deviation amount exceeds the allowable range.

At this time, whether to correct the first eye refractive power of the subject eye E to the second eye refractive power may be determined based on the ring image captured by the first measurement optical system 100 (that is, the ring image when the first eye refractive power is calculated). For example, in fitting a circle using at least three pixel positions of a ring image, when errors in shape and radius of curvature are large, the first eye refractive power may be corrected to the second eye refractive power.

In this manner, the ophthalmic apparatus of the present example corrects the measurement result of the optical characteristics (eye refractive power) of the subject eye based on the contrast information of the subject eye. For example, even when the subject eye is the intraocular lens implanted eye and the measurement results of the optical characteristics of the subject eye are not appropriately acquired, the accuracy of the measurement results can be improved by correcting the measurement results using contrast information.

In the present example, a configuration is described as an example in which contrast information with respect to the diopter information of the subject eye E is output as a graph 700, but the present invention is not limited thereto. For example, a graph of contrast information reflecting the prescriptions for the subject eye may be output. As an example, when spectacles are prescribed for the subject eye for calibration, a graph that takes into account the calibration amount may be output. More specifically, when -3.0D is added to the subject eye, the contrast value K' may be shifted by an amount corresponding to -3.0D. That is, the peak value P and the peripheral value thereof may be shifted by -3.0D. In addition, when adding an additional calibration amount to the subject eye in this manner, both clear vision regions of both eyes may be output such that the visual performance of both subject eyes can be easily understood.

In the present example, a configuration is described as an example in which the contrast measurement with respect to the subject eye E is executed after the eye refractive power measurement with respect to the subject eye E, but the present invention is not limited thereto. In the present example, only the contrast measurement for the subject eye E may be executed. In this case, by moving the pattern target plate 313 from the upper limit to the lower limit of the movable range of the pattern target plate 313, a graph 700 including the peak value of the contrast value and the peripheral value thereof can be acquired.

In the present example, a configuration is described as an example in which contrast information is acquired in a so-called eye refractive power measurement apparatus, in which the ophthalmic apparatus 10 can acquire the eye refractive power of the subject eye, but the present invention is not limited thereto. The ophthalmic apparatus 10 may be a so -called wavefront aberration measurement apparatus capable of acquiring optical characteristics of a subject eye. More specifically, the ophthalmic apparatus 10 may be a wavefront aberration measurement apparatus having an optical system for dividing and detecting a reflection light flux from the fundus with a Shack-Hartmann sensor. For example, even when the ophthalmic apparatus 10 is a wavefront aberration measurement apparatus, a graph 700 may be acquired by measuring the contrast of the subject eye E, and output.

In addition, in the present example, a configuration is described as an example in which, in the ophthalmic apparatus 10, the light source 111 of the projection optical system 110 and the light source 311 (pattern target plate 313) of the projection optical system 310 are disposed in different optical paths, and the imaging element 126 of the light receiving optical system 120 and the imaging element 322 of the light receiving optical system 320 are disposed in different optical paths, but the present invention is not limited thereto. For example, each light source may be disposed in different optical paths, and each imaging element may be arranged in the same optical path (that is, shared). In this case, a ring image for measuring eye refractive power may be projected onto the fundus of the subject eye, a pattern image for acquiring contrast information may be projected onto the fundus of the subject eye, and each image may be detected by a single imaging element.

## Claims

1. An ophthalmic apparatus (10) that objectively measures optical characteristics of a subject eye (E), comprising:
an eye refractive power measuring optical system including a first light projecting optical system (110) that projects a first measurement light flux toward a fundus of the subject eye (E), and a first light receiving optical system (120) in which a first detector detects a first reflection light flux, reflected by the fundus, of the first measurement light flux as a first index pattern image, and for acquiring an eye refractive power of the subject eye (E); and
a contrast measuring optical system including a second light projecting optical system (310) that projects a patterned second measurement light flux toward the fundus of the subject eye (E), and a second light receiving optical system (320) in which a second detector detects a second index pattern image formed by a second reflection light flux, reflected by the fundus, of the second measurement light flux, and for acquiring contrast information related to a contrast of the subject eye (E),
wherein the first detector and the second detector are different detectors.

2. The ophthalmic apparatus (10) according to claim 1,
wherein in the first light receiving optical system (120), the first detector detects the first reflection light flux, which passes through a pupil divided region formed by dividing a pupil region of the subject eye (E), as the first index pattern image, and
in the second light receiving optical system (320), the second detector detects the second index pattern image formed by the second reflection light flux which passes through the pupil region of the subject eye (E).

3. The ophthalmic apparatus (10) according to claim 1 or 2,
wherein the first light receiving optical system (120) has a pattern optical member configured to condense the first reflection light flux from the fundus as a pattern light flux on the first detector, and the pattern optical member causes the first detector to detect the first index pattern image.

4. The ophthalmic apparatus (10) of claim 3,
wherein the pattern optical member is a ring optical member configured to condense the first reflection light flux from the fundus as a ring light flux on the first detector, and the ring optical member causes the first detector to detect a ring image as the first index pattern image.

5. The ophthalmic apparatus (10) according to claim 3 or 4, further comprising:
a first optical path branching member configured to branch an optical path of the first light projecting optical system (110) and an optical path of the first light receiving optical system (120); and
a second optical path branching member configured to branch an optical path of the first light receiving optical system (120) and an optical path of the second light receiving optical system (320),
wherein the second optical path branching member is arranged between the first optical path branching member and the pattern optical member to dispose the first detector and the second detector in different optical paths.

6. The ophthalmic apparatus (10) according to any one of claims 1 to 5,
wherein the first measurement light flux is an infrared light flux and the second measurement light flux is a visible light flux.

7. The ophthalmic apparatus (10) according to any one of claims 1 to 6, further comprising:
a first acquisition means configured to project the first measurement light flux toward the fundus of the subject eye (E), and cause the first detector to detect the first reflection light flux, reflected by the fundus, of the first measurement light flux, to acquire optical characteristics of the subject eye (E);
a second acquisition means configured to project the second measurement light flux toward the fundus of the subject eye (E), and cause the second detector to detect the second reflection light flux, reflected by the fundus, of the second measurement light flux, to acquire a plurality of contrast information with at least different diopter information in the subject eye (E); and
an output means configured to output change information of the contrast information with respect to the diopter information.

8. The ophthalmic apparatus (10) according to claim 7,
wherein the change information includes first data indicating a peak value of the contrast information with respect to the diopter information.

9. The ophthalmic apparatus (10) according to claim 8,
wherein the change information includes second data indicating a peripheral value for the first data.

10. The ophthalmic apparatus (10) according to any one of claims 7 to 9,
wherein the output means is configured to output, as the change information, a graph showing a distribution of the contrast information with respect to the diopter information.

11. The ophthalmic apparatus (10) according to claim 10,
wherein the output means is configured to output, as the change information, the graph continuously showing a change in the contrast information accompanied by a change in the diopter information.

12. The ophthalmic apparatus (10) according to any one of claims 7 to 10,
wherein the second acquisition means is configured to acquire the contrast information with different diopter information of the subject eye (E), based on the optical characteristics of the subject eye (E) acquired by the first acquisition means.

13. The ophthalmic apparatus (10) according to any one of claims 7 to 11,
wherein the second acquisition means is configured to project the patterned light flux as the second measurement light flux, cause the second detector to detect the second index pattern image changing periodically and formed by the second reflection light flux, and acquire the contrast information based on luminance information in the second index pattern image.

14. The ophthalmic apparatus (10) according to any one of claims 7 to 13, further comprising:
a correction means configured to correct the optical characteristics acquired by the first acquisition means, based on the contrast information acquired by the second acquisition means.

15. An ophthalmic apparatus (10) that objectively measures optical characteristics of a subject eye (E), comprising:
a first acquisition means configured to project a first measurement light flux toward a fundus of the subject eye (E), and cause a first detector to detect a first reflection light flux, reflected by the fundus, of the first measurement light flux, to acquire optical characteristics of the subject eye (E);
a second acquisition means configured to project a second measurement light flux toward the fundus of the subject eye (E), and cause a second detector to detect a second reflection light flux, reflected by the fundus, of the second measurement light flux, to acquire a plurality of contrast information with at least different diopter information in the subject eye (E); and
an output means configured to output change information of the contrast information with respect to the diopter information.

## Patentansprüche

1. Ophthalmische Vorrichtung (10), die optische Eigenschaften eines Auges (E) eines Subjekts objektiv misst, umfassend:
ein optisches System zum Messen der Brechkraft des Auges, das ein erstes optisches Lichtprojektionssystem (110), das einen ersten Messlichtfluss in Richtung eines Fundus des Auges (E) des Subjekts projiziert, und ein erstes optisches Lichtempfangssystem (120), in dem ein erster Detektor einen ersten Reflexionslichtfluss, der von dem Fundus reflektiert wird, des ersten Messlichtflusses als ein erstes Indexmusterbild detektiert, und zum Erfassen einer Brechkraft des Auges (E) des Subjekts umfasst; und
ein optisches Kontrastmesssystem, das ein zweites optisches Lichtprojektionssystem (310), das einen gemusterten zweiten Messlichtfluss in Richtung des Fundus des Auges (E) des Subjekts projiziert, und ein zweites optisches Lichtempfangssystem (320), in dem ein zweiter Detektor ein zweites Indexmusterbild detektiert, das durch einen zweiten Reflexionslichtfluss, der von dem Fundus reflektiert wird, des zweiten Messlichtflusses gebildet wird, und zum Erfassen von Kontrastinformationen in Bezug auf einen Kontrast des Auges (E) des Subjekts umfasst,
wobei der erste Detektor und der zweite Detektor unterschiedliche Detektoren sind.

2. Ophthalmische Vorrichtung (10) nach Anspruch 1,
wobei in dem ersten optischen Lichtempfangssystem (120) der erste Detektor den ersten Reflexionslichtfluss, der durch einen geteilten Pupillenbereich, der durch Teilen eines Pupillenbereichs des Auges (E) des Subjekts gebildet wird, verläuft, als das erste Indexmusterbild detektiert, und
in dem zweiten optischen Lichtempfangssystem (320) der zweite Detektor das zweite Indexmusterbild detektiert, das durch den zweiten Reflexionslichtfluss gebildet wird, der durch den Pupillenbereich des Auges (E) des Subjekts verläuft.

3. Ophthalmische Vorrichtung (10) nach Anspruch 1 oder 2,
wobei das erste optische Lichtempfangssystem (120) ein optisches Musterelement aufweist, das konfiguriert ist, um den ersten Reflexionslichtfluss von dem Fundus als einen Musterlichtfluss auf dem ersten Detektor zu kondensieren, und das optische Musterelement den ersten Detektor veranlasst, das erste Indexmusterbild zu detektieren.

4. Ophthalmische Vorrichtung (10) nach Anspruch 3,
wobei das optische Musterelement ein optisches Ringelement ist, das konfiguriert ist, um den ersten Reflexionslichtfluss von dem Fundus als einen Ringlichtfluss auf dem ersten Detektor zu kondensieren, und das optische Ringelement den ersten Detektor veranlasst, ein Ringbild als das erste Indexmusterbild zu detektieren.

5. Ophthalmische Vorrichtung (10) nach Anspruch 3 oder 4, ferner umfassend:
ein erstes optisches Pfadverzweigungselement, das konfiguriert ist, um einen optischen Pfad des ersten optischen Lichtprojektionssystems (110) und einen optischen Pfad des ersten optischen Lichtempfangssystems (120) zu verzweigen; und
ein zweites optisches Pfadverzweigungselement, das konfiguriert ist, um einen optischen Pfad des ersten optischen Lichtempfangssystems (120) und einen optischen Pfad des zweiten optischen Lichtempfangssystems (320) zu verzweigen,
wobei das zweite optische Pfadverzweigungselement zwischen dem ersten optischen Pfadverzweigungselement und dem optischen Musterelement angeordnet ist, um den ersten Detektor und den zweiten Detektor in unterschiedlichen optischen Pfaden anzuordnen.

6. Ophthalmische Vorrichtung (10) nach einem der Ansprüche 1 bis 5,
wobei der erste Messlichtfluss ein Infrarotlichtfluss ist und der zweite Messlichtfluss ein sichtbarer Lichtfluss ist.

7. Ophthalmische Vorrichtung (10) nach einem der Ansprüche 1 bis 6, ferner umfassend:
ein erstes Erfassungsmittel, das konfiguriert ist, um den ersten Messlichtfluss in Richtung des Fundus des Auges (E) des Subjekts zu projizieren und den ersten Detektor zu veranlassen, den ersten Reflexionslichtfluss, der von dem Fundus reflektiert wird, des ersten Messlichtflusses zu detektieren, um optische Eigenschaften des Auges (E) des Subjekts zu erfassen;
ein zweites Erfassungsmittel, das konfiguriert ist, um den zweiten Messlichtfluss in Richtung des Fundus des Auges (E) des Subjekts zu projizieren und den zweiten Detektor zu veranlassen, den zweiten Reflexionslichtfluss, der von dem Fundus reflektiert wird, des zweiten Messlichtflusses zu detektieren, um eine Vielzahl von Kontrastinformationen mit mindestens verschiedenen Dioptrieninformationen in dem Auge (E) des Subjekts zu erfassen; und
ein Ausgabemittel, das konfiguriert ist, um Änderungsinformationen der Kontrastinformationen in Bezug auf die Dioptrieninformationen auszugeben.

8. Ophthalmische Vorrichtung (10) nach Anspruch 7,
wobei die Änderungsinformationen erste Daten umfassen, die einen Spitzenwert der Kontrastinformationen in Bezug auf die Dioptrieninformationen angeben.

9. Ophthalmische Vorrichtung (10) nach Anspruch 8,
wobei die Änderungsinformationen zweite Daten umfassen, die einen peripheren Wert für die ersten Daten angeben.

10. Ophthalmische Vorrichtung (10) nach einem der Ansprüche 7 bis 9,
wobei das Ausgabemittel konfiguriert ist, um als die Änderungsinformationen einen Graphen auszugeben, der eine Verteilung der Kontrastinformationen in Bezug auf die Dioptrieninformationen zeigt.

11. Ophthalmische Vorrichtung (10) nach Anspruch 10,
wobei das Ausgabemittel konfiguriert ist, um als die Änderungsinformationen den Graphen auszugeben, der kontinuierlich eine Änderung der Kontrastinformationen begleitet von einer Änderung der Dioptrieninformationen zeigt.

12. Ophthalmische Vorrichtung (10) nach einem der Ansprüche 7 bis 10,
wobei das zweite Erfassungsmittel konfiguriert ist, um die Kontrastinformationen mit unterschiedlichen Dioptrieninformationen des Auges (E) des Subjekts basierend auf den optischen Eigenschaften des Auges (E) des Subjekts, die von dem ersten Erfassungsmittel erfasst werden, zu erfassen.

13. Ophthalmische Vorrichtung (10) nach einem der Ansprüche 7 bis 11,
wobei das zweite Erfassungsmittel konfiguriert ist, um den gemusterten Lichtfluss als den zweiten Messlichtfluss zu projizieren, den zweiten Detektor zu veranlassen, das zweite Indexmusterbild zu detektieren, das sich periodisch ändert und durch den zweiten Reflexionslichtfluss gebildet wird, und die Kontrastinformationen basierend auf Luminanzinformationen in dem zweiten Indexmusterbild zu erfassen.

14. Ophthalmische Vorrichtung (10) nach einem der Ansprüche 7 bis 13, ferner umfassend:
ein Korrekturmittel, das konfiguriert ist, um die optischen Eigenschaften, die von dem ersten Erfassungsmittel erfasst werden, basierend auf den Kontrastinformationen, die von dem zweiten Erfassungsmittel erfasst werden, zu korrigieren.

15. Ophthalmische Vorrichtung (10), die optische Eigenschaften eines Auges (E) eines Subjekts objektiv misst, umfassend:
ein erstes Erfassungsmittel, das konfiguriert ist, um einen ersten Messlichtfluss in Richtung eines Fundus des Auges (E) des Subjekts zu projizieren und einen ersten Detektor zu veranlassen, einen ersten Reflexionslichtfluss, der von dem Fundus reflektiert wird, des ersten Messlichtflusses zu detektieren, um optische Eigenschaften des Auges (E) des Subjekts zu erfassen;
ein zweites Erfassungsmittel, das konfiguriert ist, um einen zweiten Messlichtfluss in Richtung des Fundus des Auges (E) des Subjekts zu projizieren und einen zweiten Detektor zu veranlassen, einen zweiten Reflexionslichtfluss, der von dem Fundus reflektiert wird, des zweiten Messlichtflusses zu detektieren, um eine Vielzahl von Kontrastinformationen mit mindestens verschiedenen Dioptrieninformationen in dem Auge (E) des Subjekts zu erfassen; und
ein Ausgabemittel, das konfiguriert ist, um Änderungsinformationen der Kontrastinformationen in Bezug auf die Dioptrieninformationen auszugeben.

## Revendications

1. Appareil ophtalmique (10) qui mesure objectivement des caractéristiques optiques d'un oeil d'un sujet (E), comprenant :
un système optique de mesure de puissance de réfraction de l'oeil incluant un premier système optique de projection de lumière (110) qui projette un premier flux lumineux de mesure vers un fond de l'oeil du sujet (E), et un premier système optique de réception de lumière (120) dans lequel un premier détecteur détecte un premier flux lumineux de réflexion, réfléchi par le fond, du premier flux lumineux de mesure en tant qu'une première image de motif d'indice, et pour acquérir une puissance de réfraction de l'oeil de l'oeil du sujet (E) ; et
un système optique de mesure de contraste incluant un second système optique de projection de lumière (310) qui projette un second flux lumineux de mesure à motif vers le fond de l'oeil du sujet (E), et un second système optique de réception de lumière (320) dans lequel un second détecteur détecte une seconde image de motif d'indice formée par un second flux lumineux de réflexion, réfléchi par le fond, du second flux lumineux de mesure, et pour acquérir des informations de contraste relatives à un contraste de l'oeil du sujet (E),
dans lequel le premier détecteur et le second détecteur sont des détecteurs différents.

2. Appareil ophtalmique (10) selon la revendication 1,
dans lequel dans le premier système optique de réception de lumière (120), le premier détecteur détecte le premier flux lumineux de réflexion, qui passe à travers une région divisée de pupille formée en divisant une région de pupille de l'oeil du sujet (E), en tant que la première image de motif d'indice, et
dans le second système optique de réception de lumière (320), le second détecteur détecte la seconde image de motif d'indice formée par le second flux lumineux de réflexion qui passe à travers la région de pupille de l'oeil du sujet (E).

3. Appareil ophtalmique (10) selon la revendication 1 ou 2,
dans lequel le premier système optique de réception de lumière (120) a un élément optique de motif configuré pour condenser le premier flux lumineux de réflexion provenant du fond en tant qu'un flux lumineux de motif sur le premier détecteur, et l'élément optique de motif amène le premier détecteur à détecter la première image de motif d'indice.

4. Appareil ophtalmique (10) selon la revendication 3,
dans lequel l'élément optique de motif est un élément optique annulaire configuré pour condenser le premier flux lumineux de réflexion provenant du fond en tant qu'un flux lumineux annulaire sur le premier détecteur, et l'élément optique annulaire amène le premier détecteur à détecter une image annulaire en tant que la première image de motif d'indice.

5. Appareil ophtalmique (10) selon la revendication 3 ou 4, comprenant en outre :
un premier élément de dérivation de chemin optique configuré pour dériver un chemin optique du premier système optique de projection de lumière (110) et un chemin optique du premier système optique de réception de lumière (120) ; et
un second élément de dérivation de chemin optique configuré pour dériver un chemin optique du premier système optique de réception de lumière (120) et un chemin optique du second système optique de réception de lumière (320),
dans lequel le second élément de dérivation de chemin optique est agencé entre le premier élément de dérivation de chemin optique et l'élément optique de motif pour disposer le premier détecteur et le second détecteur dans des chemins optiques différents.

6. Appareil ophtalmique (10) selon l'une quelconque des revendications 1 à 5,
dans lequel le premier flux lumineux de mesure est un flux lumineux infrarouge et le second flux lumineux de mesure est un flux lumineux visible.

7. Appareil ophtalmique (10) selon l'une quelconque des revendications 1 à 6, comprenant en outre :
un premier moyen d'acquisition configuré pour projeter le premier flux lumineux de mesure vers le fond de l'oeil du sujet (E), et amener le premier détecteur à détecter le premier flux lumineux de réflexion, réfléchi par le fond, du premier flux lumineux de mesure, pour acquérir des caractéristiques optiques de l'oeil du sujet (E) ;
un second moyen d'acquisition configuré pour projeter le second flux lumineux de mesure vers le fond de l'oeil du sujet (E), et amener le second détecteur à détecter le second flux lumineux de réflexion, réfléchi par le fond, du second flux lumineux de mesure, pour acquérir une pluralité d'informations de contraste avec au moins des informations dioptriques différentes dans l'oeil du sujet (E) ; et
un moyen de sortie configuré pour délivrer en sortie des informations de changement des informations de contraste par rapport aux informations dioptriques.

8. Appareil ophtalmique (10) selon la revendication 7,
dans lequel les informations de changement incluent des premières données indiquant une valeur de crête des informations de contraste par rapport aux informations dioptriques.

9. Appareil ophtalmique (10) selon la revendication 8,
dans lequel les informations de changement incluent des secondes données indiquant une valeur périphérique pour les premières données.

10. Appareil ophtalmique (10) selon l'une quelconque des revendications 7 à 9,
dans lequel le moyen de sortie est configuré pour délivrer en sortie, en tant qu'informations de changement, un graphique montrant une distribution des informations de contraste par rapport aux informations dioptriques.

11. Appareil ophtalmique (10) selon la revendication 10,
dans lequel le moyen de sortie est configuré pour délivrer en sortie, en tant qu'informations de changement, le graphique montrant en continu un changement des informations de contraste accompagné d'un changement des informations dioptriques.

12. Appareil ophtalmique (10) selon l'une quelconque des revendications 7 à 10,
dans lequel le second moyen d'acquisition est configuré pour acquérir les informations de contraste avec des informations dioptriques différentes de l'oeil du sujet (E), sur la base des caractéristiques optiques de l'oeil du sujet (E) acquises par le premier moyen d'acquisition.

13. Appareil ophtalmique (10) selon l'une quelconque des revendications 7 à 11,
dans lequel le second moyen d'acquisition est configuré pour projeter le flux lumineux à motif en tant que le second flux lumineux de mesure, amener le second détecteur à détecter la seconde image de motif d'indice changeant périodiquement et formée par le second flux lumineux de réflexion, et acquérir les informations de contraste sur la base d'informations de luminance dans la seconde image de motif d'indice.

14. Appareil ophtalmique (10) selon l'une quelconque des revendications 7 à 13, comprenant en outre :
un moyen de correction configuré pour corriger les caractéristiques optiques acquises par le premier moyen d'acquisition, sur la base des informations de contraste acquises par le second moyen d'acquisition.

15. Appareil ophtalmique (10) qui mesure objectivement des caractéristiques optiques d'un oeil d'un sujet (E), comprenant :
un premier moyen d'acquisition configuré pour projeter un premier flux lumineux de mesure vers un fond de l'oeil du sujet (E), et amener un premier détecteur à détecter un premier flux lumineux de réflexion, réfléchi par le fond, du premier flux lumineux de mesure, pour acquérir des caractéristiques optiques de l'oeil du sujet (E) ;
un second moyen d'acquisition configuré pour projeter un second flux lumineux de mesure vers le fond de l'oeil du sujet (E), et amener un second détecteur à détecter un second flux lumineux de réflexion, réfléchi par le fond, du second flux lumineux de mesure, pour acquérir une pluralité d'informations de contraste avec au moins des informations dioptriques différentes dans l'oeil du sujet (E) ; et
un moyen de sortie configuré pour délivrer en sortie des informations de changement des informations de contraste par rapport aux informations dioptriques.
